# EUROPEAN PATENT SPECIFICATION

(11) **EP 4 167 755 B1**
(45) Date of publication and mention of the grant of the patent: **25.03.2026**
(21) Application number: 21736287.0
(22) Date of filing: 23.06.2021
(51) Int. Cl.: A23L 19/00, A23L 19/12, A23L 33/135, A23L 9/10, A23J 1/16, A23L 2/08, C07K 1/34, B01D 61/14, A23L 33/185

(54) **METHOD FOR SEPARATION OF POTATO PROTEINS FROM PHENOLIC AND/OR GLYCOALKALOID COMPOUNDS**
VERFAHREN ZUR ABTRENNUNG VON KARTOFFELPROTEINEN AUS PHENOLISCHEN UND/ODER GLYCOALKALOIDVERBINDUNGEN
PROCÉDÉ DE SÉPARATION DE PROTÉINES DE POMME DE TERRE DE COMPOSÉS PHÉNOLIQUES ET/OU GLYCOALCALOÏDES

(30) Priority: 23.06.2020 DK PA202070411; 27.11.2020 DK PA202070792
(43) Date of publication of application: 26.04.2023
(73) Proprietor: Coöperatie Koninklijke Cosun U.A., 4814 NE Breda (NL)
(72) Inventor: LIHME, Allan Otto Fog, 3520 Farum (DK); HANSEN, Marie Bendix, 2000 Frederiksberg (DK); LINDVED, Bodil Kjær, 3060 Espergærde (DK); JORDENS, Rick Adrianus Petrus, 6613AS Balgoij (NL)
(74) Representative: Nederlandsch Octrooibureau
(86) International application number: PCT/EP2021/067206
(87) International publication number: WO 2021/260038

(56) References cited:
- WO-A1-02/060273
- WO-A1-2019/057257
- WO-A1-2019/215153
- US-A1- 2003 077 265
- US-A1- 2019 082 715
- US-A1- 2019 274 332
- SISSEL LOKRA ET AL: "Industrial Proteins from Potato Juice. A Review", FOOD, vol. 3, no. 1, 1 January 2009 (2009-01-01), pages 88 - 95, XP055434942
- KNUT OLAV STRÃ TKVERN ET AL: "Recovery of Native Potato Protein Comparing Expanded Bed Adsorption and Ultrafiltration", FOOD AND BIOPROCESS TECHNOLOGY ; AN INTERNATIONAL JOURNAL, SPRINGER-VERLAG, NEW YORK, vol. 5, no. 5, 15 January 2011 (2011-01-15), pages 1939 - 1949, XP035067433, ISSN: 1935-5149, DOI: 10.1007/S11947-010-0494-2

## Description

### TECHNICAL FIELD OF THE INVENTION

The present invention relates to methods for isolating compounds, particularly functional proteins and phenolic and/or glycoalkaloid compounds, from potato fruit juice or derivatives thereof, as defined in the appended claims.

### BACKGROUND OF THE INVENTION

Many biological fluids and extracts contain soluble proteins and soluble phenolic compounds such as phenolic acids, flavonoids and tannins. This is particularly the case for liquid plant extracts and extracts of *e.g.* yeast and algae. Many of the phenolic compounds are highly reactive and may *e.g.* be oxidized to create oligomers and polymers. Such phenolic compounds may adsorb to, and even react covalently, with proteins. The susceptibility of phenolic compounds towards adsorption and/or their covalent reaction with proteins is influenced by many physico-chemical parameters, such as the degree of oxidation, pH, temperature and time of contact.

The potato *(Solanum tuberosum* L.) is a tuber that is largely used as food or in food applications and is a source of different bioactive compounds, such as starch, dietary fiber, amino acids, minerals, vitamins, glycoalkaloid compounds and phenolic compounds.

Potato fruit juice is a complex mixture of soluble and insoluble material comprising proteins, starch, minerals, (toxic) glycoalkaloids, (insoluble) fibers and monomeric and polymeric reactive phenols.

Due to oxidation of natural phenolic compounds, potato fruit juice may turn brown or black. Chemically, the phenolic compounds are oxidized into quinones, which combine into a dark polymer residue. During the oxidation process, the potato proteins may undergo reaction and partial crosslinking. This crosslinking may dramatically reduce the solubility of the potato proteins. Thus, from a technological point of view, the complexity and instability of the potato fruit juice makes the separation and isolation of native potato proteins much more complicated and economically demanding than the isolation of proteins from other types of protein solutions.

Purification of potato proteins from potato fruit juice is further complicated by the presence of glycoalkaloids, which must be removed before the potato proteins may be used in human nutrition. Glycoalkaloids are well known anti-nutritional factors. The glycosylated forms of glycoalkaloids, such as α-solanine and α-chaconine, show the highest toxicity. The aglycons, such as solanidine, have a more than 100-fold lower liver toxicity. α-Solanine and α-chaconine make up more than 95 % of the total glycoalkaloid content in potatoes. Other glycoalkaloids are for example tomatine, tomatidenol and demissidine. Glycoalkaloids have a bitter taste and negatively affect many of the physical and/or biological properties of potato proteins. For food applications, the taste threshold of glycoalkaloids is about 140-170 mg of glycoalkaloids per kg of product. This threshold strongly limits the applications of known native potato protein isolates in foods.

Glycoalkaloids are known to possess antimicrobial and pesticidal properties which may be exploited in various types of products, such as for example in biological crop protection products. Moreover, beneficial bioactivities to human health, such as antibacterial, antiinflammatory and antiobesity effects have been attributed to glycoalkaloids and their use as anti-cancer agents, and their use in vaccine adjuvants have been described. See in this respect B. Nepal et al., Processes, 2019, 7, pp 513- 538 and S.L. Sampaio et al., Trends in Food Science & Technology, 2020, 103, pp 118-129. As will be appreciated by those skilled in the art, these applications require the glycoalkaloids to be available in an (highly) enriched natural fraction.

Phenolic compounds are synthetized by the potato plant as a protection response against bacteria, fungi, viruses and insects. Several publications suggest that phenolic compounds from potato exhibit health-promoting effects in humans. See in this respect, H. Akyol et al., Int. J. Mol. Sci., 2016, 17, pp 835-854.

Several compounds, such as proteins and metabolites, comprised in potatoes are valuable and useful in many different applications, such as in nutrition, medical treatments, cosmetics and as acceptable process aids for industrial manufacture of the same. Particularly, patatin and protease inhibitor proteins and phenolic compounds in potato fruit juice or derivatives thereof (waste products of the potato starch manufacturing industry) are interesting and become even more valuable and useful in isolated or more pure form. Patatin and protease inhibitor proteins and phenolic compounds are highly nutritious and have many desirable functional characteristics which could make them useful in both food and industrial products.

Phenolic compounds from potato may constitute valuable products for a variety of applications, *e.g.* as antioxidants, anti-cancer agents and antimicrobial agents. Phenolic compounds present in potatoes are phenolic acids and flavonoids including flavonols, flavanols, and anthocyanins.

Phenolic acids are the most abundant phenolic compounds in potatoes. Chlorogenic acid, which is the ester of caffeic acid and quinic acid, constitutes 90% of the phenolic compounds in potato peels and exists in the form of three main isomers, chlorogenic acid (5-O-caffeoylquinicacid), neochlorogenic acid (3-O-caffeoylquinic acid), and cryptochlorogenic acid (4-O-caffeoylquinic acid). Also, caffeic acid is quantified at 25-72 mg/100 g in potatoes by many researchers. Other phenolic acids such as ferulic acid, gallic acid, and *p*-coumaric acid have also been quantified in potatoes, ranging from 0- 5 mg/100 g dry weight. Furthermore, syringic acid, vanillic acid, sinapic acid, and salicylic acid are present in small quantities (see H. Akyol et al., Int. J. Mol. Sci., 2016, 17, pp 835-854).

The six significant subclasses of flavonoids are the flavones, flavonols, flavanones, flavan-3-ols, anthocyanidins, and isoflavones. Occasionally they can be found as aglycones but most flavonoids are attached to sugars (glycosides). In potatoes, one of the most abundant flavonoids is catechin, ranging between 0 and 204 mg/100 g dry weight. Flavonols, like quercetin and kaempferol rutinose, are also present in potato tubers. Flavonoids are typically present in an amount of more than 30 mg per 100 g fresh weight in white fleshed potatoes and this level is nearly doubled in red and purple fleshed potatoes as a result of anthocyanins. The most common anthocyanidins (the deglycosylated forms of anthocyanins) present in potatoes are malvidin, petunidin, delphinidin, and peonidin in purple tubers and pelargonidin in red ones. In addition to this anthocyanin, aglycones, cyanidin, and petanin are also found in potatoes (see H. Akyol et al., Int. J. Mol. Sci., 2016, 17, pp 835-854).

Native/soluble or at least partially native/soluble patatin and/or protease inhibitor proteins, *i.e.* not fully denatured or coagulated potato proteins, may be used as techno-functional ingredients in the preparation of foodstuffs, for example to provide (thermo)gelling, foaming, water-binding or emulsification properties. For these purposes, the proteins preferably are devoid of compounds that may give rise to unwanted color and taste formation and/or influence the quality and stability of the prepared food.

Thus, separation or isolation of native/soluble proteins and phenolic and/or glycoalkaloid compounds from potato fruit juice or a derivative thereof is important for obtaining the necessary purity and quality of these valuable compounds. Potato fruit juice typically contains phenolic and glycoalkaloid compounds. As will be appreciated by those skilled in the art, the availability of an efficient process resulting in the separation of a stream enriched in phenolic and glycoalkaloid compounds from potato fruit juice is also valuable because techniques are available in the art for the separation of phenolic compounds from glycoalkaloid compounds such as chromatography, including ion-exchange chromatography and hydrophobic interaction chromatography. In this respect, reference is made to A.F. Sánchez Maldonado et al., Extraction and fractionation of phenolic acids and glycoalkaloids from potato peels using acidified water/ethanol-based solvents, Food Research International, 2014, 65, pp 27-34, and to R. Shakya et al., Rapid screening of ascorbic acid, glycoalkaloids, and phenolics in potato using high-performance liquid chromatography, J. Agric. Food Chem., 2006, 54, pp 5253-5260.

Membrane filtration, in particular cross-flow ultrafiltration and diafiltration, is a well-known method for separation of high molecular weight compounds, like proteins, from low molecular weight compounds like small carbohydrates, organic acids and minerals (salts). A widespread example of this technique is the manufacture of whey protein concentrates and whey protein isolates from cheese whey by combined ultrafiltration and diafiltration.

Diafiltration, in the context of the present invention, is a technique that uses membranes to completely remove, replace, or lower the concentration of salts and/or other low molecular weight substances from solutions containing proteins, peptides, nucleic acids, and other high molecular weight biomolecules. The process selectively utilizes permeable (porous) membrane filters to separate the components of solutions and suspensions based on their molecular size. An ultrafiltration membrane retains molecules that are larger than the pores of the membrane while smaller molecules such as salts, phenolic and/or glycoalkaloid compounds, solvents and water, may pass through the membrane. In a diafiltration process water is added to the retentate, while the membrane filtration process continuously removes water, salts and low molecular weight compounds to the permeate side of the membrane.

B. Dutré and G. Trägårdh, Macrosolute-microsolute separation by ultrafiltration: A review of diafiltration processes and applications, Desalination, 1994, 95, pp 227-267, disclose a review of the theory and applications of diafiltration in several commercial scale examples.

WO97/42834A1 describes in Example 1 a process for the production of undenatured potato protein from potato fruit juice. The potato fruit juice was pretreated causing flocculation of contaminations and subjected to disc stack centrifuging to remove flocculates, other solids and microorganisms. The disc stack centrifugation necessarily removes substantially all of the insoluble fibers present in the potato fruit juice. The supernatant obtained after centrifugation was concentrated about 12 times by ultrafiltration using a continuous ultrafiltration unit equipped with polyethersulfone membranes, cut-off value 5 kDa. The retentate was washed by means of diafiltration in the presence of bisulphite to minimise polyphenol oxidation. Diafiltration was continued until salts and metal concentrations had reached acceptable levels. The concentrate was freeze-dried until a dry solid content of 92% was reached. The freeze-dried powder contained 70% protein and no detectable levels of glycoalkaloids. WO97/42834A1 does not comment on the removal of polyphenols from the product.

H.J. Zwijnenberg et al., Desalination, 144, 2002, pp 331-334, also disclose examples wherein native protein is recovered from potato fruit juice by ultrafiltration and subsequent ultrafiltration. It is described in Zwijnenberg that the potato fruit juice applied contains everything except the starch and fiber. A pretreatment was performed prior to ultrafiltration to reduce the amount of fibers. Diafiltration with tap water was carried out in order to wash out low molecular substances like amino-acids, phenolic components, glycoalkaloids and potassium. The resulting protein was spray-dried in order to get a stable and dry product that could be transported and stored during longer periods.

WO02/060273A1 discloses a process for preparing from a protein source containing phenolic compounds a preparation of water-soluble proteins having a reduced content of phenolic compounds, comprising the step of precipitating the protein by bringing the protein source into a mixture of an aqueous medium and at least a water-miscible organic solvent, such that substantially no denaturation of the protein occurs, while the pH of the mixture deviates at most 1 pH value from the isoelectric point.

WO2019/215153A1 discloses a process for separation of one or more potato proteins from a group of impurities, amongst which is phenols, in an aqueous solution, said process comprising the steps of:
a) providing an aqueous solution comprising the one or more potato proteins and a group of impurities;
b) optionally subjecting the aqueous solution to a pretreatment to clarify and remove suspended non-soluble matter;
c) precipitating the one or more potato proteins to create a suspension of precipitated potato protein in the solution containing impurities;
d) subjecting the suspension to a membrane filtration process wherein at least one member of the group of impurities passes the membrane as a permeate and the one or more precipitated potato proteins are concentrated in the retentate;
e) optionally diafiltering the retentate with one or more solvents to further remove impurities into the permeate;
f) optionally re-solubilizing the precipitated one or more potato proteins; and
g) optionally separating the group of impurities in said permeate into at least two individual fractions.

US2003/077265A1 concerns an ultrafiltration method for the isolation and purification of a proteinase inhibitor extract from potato tubers. The extraction and isolation of the proteinase inhibitor from potatoes begins with the addition of an organic acid, such as formic acid, and a salt to raw potatoes. Undesired proteins are denatured using heat treatment and removed, resulting in an extract solution suitable for ultrafiltration. The ultrafiltration process comprises concentration and diafiltration.

However, for certain separation tasks, such as the separation of proteins from phenolic and/or glycoalkaloid compounds, and in particular the separation of soluble proteins from phenolic and/or glycoalkaloid compounds in plant extracts, the membrane filtration technology is not a complete and cost-efficient process, which may be due to the tendency of phenolic and/or glycoalkaloid compounds to adhere to other compounds such as proteins, polysaccharides as well as the membrane surface.

For example, according to several scientific reports (see *e.g.* K.O Straetkvern and J.G. Schwarz, Recovery of Native Potato Protein Comparing Expanded Bed Adsorption and Ultrafiltration, Food and Bioprocess. Technol., 5(5), 2012, pp 1939-1949 and H.J. Zwijnenberg et al., Desalination, 144, 2002, pp 331-334), ultrafiltration has low selectivity and only poorly separates polyphenols and brown polyphenol complexes from proteins, thus giving a powder with a final brown hue and higher content of chlorogenic acids. Moreover, membrane concentration of potato fruit juice often leads to fouling of the membranes with corresponding low flux rates, low system productivity and a short membrane lifetime. Both disclosures describe the use of water for the applied diafiltration step. In Zwijnenberg *et al.* it is illustrated how the permeate flux decreases from the onset of diafiltration using water as the diafiltration solvent.

It is therefore an object of the invention to provide improved methods for the separation of (a) native/soluble proteins and (b) phenolic and/or glycoalkaloid compounds from potato fruit juice or derivatives thereof at industrial scale.

It is another object of the invention to provide products enriched in phenolic and glycoalkaloid compounds with sufficient quality at industrial scale.

It is yet another object of the invention to provide valuable phenolic and/or glycoalkaloid compounds with sufficient purity and quality at industrial scale.

### SUMMARY OF THE INVENTION

The present inventors have unexpectedly found that one or more of the objectives can be met by subjecting potato fruit juice or derivatives thereof to a membrane filtration process at industrial scale using diafiltration solvents with an increased ionic strength, which leads to a higher permeability of the membrane for the phenolic and/or glycoalkaloid compounds and a higher average permeate flux during the diafiltration step. The scope of the present invention is defined in the appended claims.

Accordingly, in a first aspect, the present invention provides a method for the separation of potato proteins from one or more first salts and phenolic and/or glycoalkaloid compounds in potato fruit juice or a derivative thereof, said method comprising the steps of:
(i) providing a potato fruit juice or a derivative thereof comprising potato proteins, one or more first salts and phenolic and/or glycoalkaloid compounds;
(ii) subjecting said potato fruit juice or the derivative thereof to a first cross-flow membrane filtration process wherein at least a portion of the one or more first salts and at least a portion of the phenolic and/or glycoalkaloid compounds migrate across the membrane into a first permeate and the potato proteins are retained in a first retentate;
(iii) subjecting the first retentate of step (ii) to a diafiltration step comprising adding one or more second salts and water to the first retentate while continuing the membrane filtration process to create a diafiltrate containing at least a portion of said phenolic and/or glycoalkaloid compounds and the added second salts and a retentate; and
(iv) subjecting the first permeate and/or said diafiltrate from said first cross-flow membrane filtration process to a second cross-flow membrane filtration process wherein at least a portion of the salts present therein migrate across the membrane into a second permeate and the phenolic and/or glycoalkaloid compounds are retained in a second retentate.

### DEFINITIONS

The term '*potato fruit juice or derivatives thereof'* means the liquid stream after the (industrial scale) separation of potato starch from potatoes (*solanum tuberosum* L.) and any aqueous streams derived from processing of such liquid stream by for example flocculation of contaminants and centrifugation, provided that the *'derivatives thereof'* still comprise protein, phenolic and/or glycoalkaloid compounds and (first) salts. The term further includes any aqueous stream that may be obtained by disintegration, *e.g.* by grinding, shredding and/or pressing, of the raw potatoes, peels and cuts from raw potatoes with or without extraction by addition of water or an aqueous extraction solution in combination with physical disruption of the plant tissue and/or cells. The potato fruit juice may also be obtained from potatoes, peels and cuts that have been subjected to a heating process and/or have been subjected to freezing.

The term *'potato protein(s)'* means protein or proteins present in the tubers of the potato plant *solanum tuberosum* L., particularly patatin and/or protease inhibitor.

The term *'functional proteins'* means proteins that (still) have a high level of their intrinsic techno-functional properties, such as aqueous solubility, the ability to form gels when heated in solution (thermo-gelling), the ability to create foams when aqueous solutions of the protein are whipped with air, or the ability to create emulsions when mixed with lipids in aqueous solutions of the protein. The term *'functional proteins'* as used herein is therefore considered similar to the terms *'soluble proteins', '(substantially) native proteins'* and *'(substantially) undenatured proteins'* as used in the art.

The term *'insoluble fibers'* means substances present in the potato fruit juice or in the derivatives thereof that can be separated from the liquid phase by centrifugation in a laboratory centrifuge at 4000 rpm for 30 minutes at room temperature. The precise chemical composition of the insoluble fibers may vary broadly, but may typically comprise insoluble polysaccharides, pectinates, starches and proteins and insoluble complexes of one or more of these substances.

The term *'absorbance at 600 nm'* or *'O.D.600'* as used herein relates to the amount of light of wavelength 600 nm passing through a liquid sample when measured in a spectrophotometer using 10 mm light path cuvettes. The absorbance of a liquid sample is often expressed as O.D. 600 nm. For the purpose of this disclosure, the absorbance is determined by spectrophotometry on samples diluted in 0.05 M potassium phosphate at pH 7.0, wherein, if need be, the pH is adjusted to 7.0 with sodium hydroxide or hydrochloric acid, to a read out in the range of 0.5 to 1.0, and this read out is multiplied with the dilution factor to determine the absorbance of the undiluted sample. If a sample has an absorbance of less than 1.0, no (substantial) dilution is performed, but the pH is nevertheless adjusted to 7.0 with sodium hydroxide or hydrochloric acid. In other words, the *'absorbance at 600 nm'* and *'O.D. 600'* as used herein correspond to the O.D.600 value of a sample as measured by spectrophotometry using 10 mm light path cuvettes on samples diluted in 0.05 M potassium phosphate at pH 7.0 to a read out in the range of 0.5 to 1.0 and the read out is multiplied with the dilution factor to determine the absorbance of the undiluted sample.

The term *'patatin',* also denoted herein as *'PA',* means storage glycoproteins found in potatoes (*Solanum tuberosum* L.). Patatin represents a group of immunologically identical glycoprotein isoforms with molecular masses in the range of 40-43 kDa. Patatin also has phospolipase activity capable of cleaving fatty acids from membrane lipids. For purposes of the invention, PA may be determined by different known assays, including SDS-PAGE combined with scanning densitometry (*e.g.* using a GS-900^{™} Calibrated Densitometer from BIO-RAD Laboratories, USA) including all protein bands in the molecular weight region between 35 kDa and 60 kDa in the PA category, ELISA testing using patatin specific antibodies, as well as enzymatic assays specific for the phospholipase activity (see e.g. M. Jiménez-Atiénzar et al., Determination of the phospholipase activity of patatin by a continuous spectrophotometric assay, Lipids, 2003, 38(6), pp 677-682).

The term *'protease inhibitor',* also denoted herein as *'PI',* means potato proteins, which possess molecular weights ranging from about 3 kDa to about 35 kDa, and which are capable of inhibiting the activity of *e.g.* serine proteases, cysteine proteases, aspartate proteases, and metalloproteases. For purposes of the invention PI may be determined by different known assays, including SDS-PAGE combined with scanning densitometry (*e.g.* using a GS-900^{™} Calibrated Densitometer from BIO-RAD Laboratories, USA) including all protein bands in the molecular weight region between 3 kDa and 35 kDa in the PI category, and more broadly by enzyme inhibition assays as generally described in the art (see e.g. R.E.J. Spelbrink et al., Quantitative Determination of Trypsin Inhibitory Activity in Complex Matrices, The Open Food Science Journal, 2011, 5, pp 42-46).

The term *'polyphenol oxidase',* also denoted herein as *'PPO',* means in the context of the present invention potato protein. Polyphenol oxidase (tyrosinase) (TY) is a bifunctional, copper-containing oxidase having both catecholase and cresolase activity. PPO causes the rapid polymerization of *o*-quinones to produce black, brown or red pigments (polyphenols) which cause fruit browning. The amino acid tyrosine contains a single phenolic ring that may be oxidised by the action of PPOs to form *o*-quinone. Hence, PPOs may also be referred to as tyrosinases. The catalytic action of PPO has a negative impact on the quality of several fruit and vegetable crops and results in alteration of color, flavor, texture, and nutritional value. It is a limiting factor in the handling and technological processing of crops as peeled, sliced, bruised or diseased tissues rapidly undergo browning. For purposes of the invention, PPO may be determined by different known assays as reviewed in: R. Yoruk et al., Physicochemical properties and function of plant polyphenol oxidase: A review, Journal of Food Biochemistry, 27(5), 2003, pp 361- 422.

The term *'lipoxygenase',* also denoted herein as *'LipO',* means in the context of the present invention potato proteins capable of catalyzing the dioxygenation of polyunsaturated fatty acids. Lipoxygenases have food-related applications in bread making and aroma production but they also have negative implications for the color, off-flavour and antioxidant status of plant-based foods. In potatoes (*Solanum tuberosum* L.), lipoxygenase has a molecular weight of approximately 97 kD and can be detected by SDS-PAGE (see e.g. G. Bauw et al., Patatins, Kunitz protease inhibitors and other major proteins in tuber of potato cv. Kuras, FEBS Journal, 2006, 273, pp 3569-3584). For purposes of the invention LipO may be determined by different known assays, including SDS-PAGE combined with scanning densitometry (*e.g.* using a GS-900^{™} Calibrated Densitometer from BIO-RAD Laboratories, USA) as well as enzyme activity assays as described in *e.g.* G.E. Anthon et al., Colorimetric method for the determination of lipoxygenase activity, J. Agric. Food Chem., 2001, 49, pp 32-37.

The term *'glycoalkaloids'* or *'alkaloid glucosides'* means a family of potentially toxic chemical compounds derived from alkaloids to which sugar groups are attached. Prototypical glycoalkaloids present in potatoes *(Solanum tuberosum* L.) are α-solanine and α-chaconine. In the context of the present disclosure, the level of *'total glycoalkaloids'* is expressed as the sum of α-solanine and α-chaconine. For purposes of the invention, glycoalkaloids may be determined by different known assays, including a standard HPLC assay as described in V. Alt et al., Optimization of glycoalkaloid analysis for use in industrial potato fruit juice downstreaming, Eng. Life Sci., 2005, 5, pp 562-567.

The term *'first salts'* means salts that are present in the liquid of the potato fruit juice of the derivative thereof at the outset of the separation process. Accordingly, the first salts include *'natural salts'* which are naturally present in potato fruit juice.

The term *'second salts'* concerns one or more salts added with water during the diafiltration step to increase the conductivity of the diafiltration liquid.

The term *'phenolic compounds'* means aromatic or heteroaromatic compounds comprising one or more ring systems and one or more phenolic hydroxyl groups. Phenolic compounds from potatoes include phenolics acids, flavonoids, tannins, stilbenes and lignans.

The term *'true protein concentration'* means the amount of protein per liter of a sample, calculated as the total weight or mass of amino acids per liter as determined by measuring the total nitrogen in a sample by the method of Kjeldahl and using the conversion factor N x 6.25. All samples are dialyzed against demineralized water in dialysis tubing cellulose membrane (Sigma-Aldrich, USA, cat. No.: D9652) to remove any free amino acids and low molecular weight peptides prior to the nitrogen determination.

The term *'dry weight'* as used in the context of the present invention means the weight or mass of a substance remaining after removal of water by heating at 110°C until the weight does not change anymore. The dry weight per ml sample is thus the remaining weight or mass of a sample of a substance having a volume of 1 ml before drying after removal of water by heating at 110°C until the weight does not change anymore.

The term *'soluble'* as used in the context of the present invention refers to solubility of compounds in water at a concentration of at least 1 g/L at 25 °C.

The term *'diafiltration'* in the context of the present invention means a technique that uses membranes, typically ultrafiltration membranes, to completely remove, replace, or lower the concentration of salts or other low molecular weight substances from solutions containing proteins, peptides, nucleic acids, and other high molecular weight molecules. The process selectively utilizes permeable (porous) membrane filters to separate the components of solutions and suspensions based on their molecular size. An ultrafiltration membrane retains molecules that are larger than the pores of the membrane, while smaller molecules such as salts, polyphenols, solvents and water, may pass through the membrane. In a diafiltration process, water or a buffer composition (*i.e.* the diafiltration liquid) is continuously added to the retentate while the membrane filtration process continuously removes water, salts and low molecular weight compounds to the permeate side of the membrane.

The term *'nanofiltration'* means a membrane filtration-based method that uses nanometer sized through-pores across the membrane. Nanofiltration membranes typically have pore sizes from 1-10 nanometers, smaller than that used in microfiltration and ultrafiltration, but just larger than that used in reverse osmosis. Nanofiltration membranes are typically defined by the molecular weight cut-off (MWCO) of the membrane used. The nanofiltration membranes typically have a molecular weight cut-off of between 100 and 1000 Da. Nanofiltration is applied in cross-flow mode under increased pressure.

The term *'ultrafiltration'* means a variety of membrane filtration processes in which forces like pressure or concentration gradients lead to a separation through a semipermeable membrane. Suspended solids and solutes of high molecular weight are retained in the so-called retentate, while water and low molecular weight solutes pass through the membrane in the permeate (filtrate). This separation process is typically used in industry and research for purifying and concentrating macromolecular solutions, especially protein solutions. Ultrafiltration membranes are defined by the pore size or by the molecular weight cut-off (MWCO) of the membrane used. Ultrafiltration membranes typically have a pore size of 0.01 - 0.1 µm or a molecular weight cut-off of between 1 and 500 kDa, such as between 5 and 50 kDa. Ultrafiltration is applied in cross-flow or dead-end mode. In a continuous process, ultrafiltration is applied in cross-flow mode.

The term *'microfiltration'* means a separation technique for removing micron-sized particles, like bacteria, suspended solids and colloid particles from liquid solutions. The process uses membrane filters with pores in the approximate size range of 0.1 to 10 µm, which are permeable to the fluid and dissolved substances, but retain certain particles, thus causing separation. Microfiltration membranes are defined by the nominal pore size of the membrane. Microfiltration is applied in cross-flow or dead-end mode. In a continuous process, microfiltration is applied in cross-flow mode.

The term *'turbidity'* means the measure of relative clarity of a liquid. It is an optical characteristic of water and is a measurement of the amount of light that is scattered by material in the water when light is shined through the water sample. The higher the intensity of scattered light, the higher the turbidity. Material that causes water to be turbid include clay, silt, very tiny particles of inorganic and organic matter and colloid fibers. Turbidity may be measured with a nephelometer. The units of turbidity from a calibrated nephelometer are called Nephelometric Turbidity Units (NTU).

The term *'mineral acids'* means inorganic acids, preferably the acids hydrochloric acid, nitric acid, phosphoric acid and sulfuric acid.

The term *'comprise'* and *'include'* as used throughout the specification and the accompanying items/claims as well as variations such as *'comprises', 'comprising', 'includes'* and *'including'* are to be interpreted inclusively. These words are intended to convey the possible inclusion of other elements or integers not specifically recited, where the context allows, unless specified otherwise.

The articles *'a'* and *'an'* are used herein to refer to one or to more than one (*i.e.* to one or at least one) of the grammatical object of the article. By way of example, *'an element'* may mean one element or more than one element, unless specified otherwise.

### BRIEF DESCRIPTION OF THE FIGURES

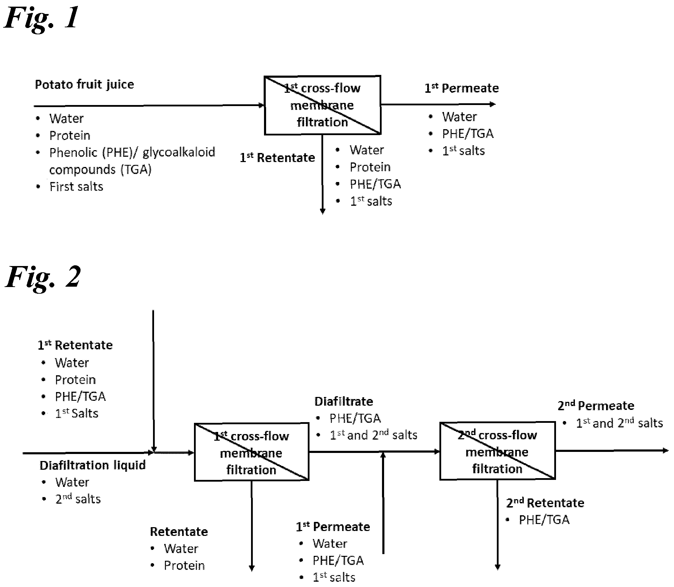

Figure 1 depicts a flow diagram of steps (i) and (ii) of the process according to the invention wherein potato fruit juice is subjected to a first cross-flow membrane filtration step, resulting in a first permeate and a first retentate.
Figure 2 depicts a flow diagram of an embodiment of steps (iii) and (iv) of the process according to the invention wherein the first retentate obtained in step (ii) is subjected in step (iii) to diafiltration using a diafiltration liquid and the combined diafiltrate and first permeate is subjected in step (iv) to a second cross-flow membrane filtration step, resulting in a second retentate enriched in phenolic and glycoalkaloid compounds and a second permeate comprising salts.
Figure 3 depicts a flow diagram of a preferred embodiment of the invention wherein steps (iii) and (iv) are followed by a step (v) of recycling the second permeate comprising salts to the diafiltration liquid.
Figure 4 depicts SDS-PAGE analysis results of potato fruit juice, diafiltration permeates and the retentate after diafiltration obtained using a process according to the invention.
Figure 5 depicts esterase activity in potato protein retentate samples that were treated at different pH and temperature using a process according to the invention.

### DETAILED DESCRIPTION

### Method for separation of potato proteins from salts and phenolic compounds

In a first aspect, the present invention relates to a method for the separation of potato proteins from one or more first salts and phenolic and/or glycoalkaloid compounds in potato fruit juice or a derivative thereof, said method comprising the steps of:
(i) providing a potato fruit juice or a derivative thereof comprising proteins, one or more first salts and phenolic and/or glycoalkaloid compounds;
(ii) subjecting said potato fruit juice or the derivative thereof to a first cross-flow membrane filtration process wherein at least a portion of the one or more first salts and at least a portion of the phenolic and/or glycoalkaloid compounds migrate across the membrane into a first permeate and the potato proteins are retained in a first retentate;
(iii) subjecting the first retentate of step (ii) to a diafiltration step comprising adding one or more second salts and water to the first retentate while continuing the membrane filtration process to create a diafiltrate containing at least a portion of said phenolic and/or glycoalkaloid compounds and the added second salts and a retentate; and
(iv) subjecting the first permeate and/or said diafiltrate from said first cross-flow membrane filtration process to a second cross-flow membrane filtration process wherein at least a portion of the salts present therein migrate across the membrane into a second permeate and the phenolic and/or glycoalkaloid compounds are retained in a second retentate.

As will be appreciated by those skilled in the art, the wording *'comprisingproteins, one or more first salts and phenolic and*/*or glycoalkaloid compounds'* as used throughout the description means that proteins and the one or more first salts are always present whereas at least one of the phenolic and glycoalkaloid compounds needs to be present.

In a preferred embodiment, the process as defined herein is performed at a temperature in the range of 1 to 60 °C, such as 5 to 55 °C, 10 to 50 °C, 15 to 48 °C, 12 to 45 °C, 18 to 30 °C, or 22 to 28 °C.

In another preferred embodiment, the first retentate remains at a temperature in the range of 1 to 60 °C, such as 5 to 55 °C, 10 to 50 °C, 15 to 48 °C, 12 to 45 °C, 18 to 30 °C, or 22 to 28 °C during step (ii) and step (iii).

In a preferred embodiment, said potato proteins, first salts and said phenolic compounds are in solution in said potato fruit juice.

In a very preferred embodiment, at least part of, more preferably all of the potato proteins remain in solution during the process steps as defined herein.

In process step (iv), the phenolic and/or glycoalkaloid compounds are separated from the one or more first salts present in the first permeate and/or the added salts in the diafiltrate such that the phenolic and/or glycoalkaloid compounds may be isolated in a purified state or a product enriched in the phenolic and glycoalkaloid compounds is obtained as a product of higher value.

In a preferred embodiment, step (iv) comprises subjecting the first permeate and the diafiltrate from said first cross-flow membrane filtration process to a second cross-flow membrane filtration process.

As a further highly important cost-saving step, the salts thus separated from the phenolic and/or glycoalkaloid compounds may then be recycled for use in the diafiltration step (iii). By doing so, there is not only saved a significant amount of water and salts for the process, but there will also be cost savings related to disposure of waste.

Accordingly, in a very preferred embodiment, the addition of the one or more added second salts and water in step (iii) is fully, or predominantly, performed using the one or more first salts and water separated from phenolic and/or glycoalkaloid compounds in process step (iv).

In a preferred embodiment, the second permeate obtained in step (iv) is used, at least in part, as the source of said one or more second salts and water in step (iii). In other words, in a preferred embodiment the addition of one or more second salts and water of step (iii) is fully, or predominantly, performed using the one or more first salts and water present in the second permeate that is separated from phenolic and/or glycoalkaloid compounds in step (iv).

In a preferred embodiment, step (iv) comprises subjecting the first permeate from said first cross-flow membrane filtration process obtained in step (ii) to a second cross-flow membrane filtration process wherein at least a portion of the salts present therein migrate across the membrane into a second permeate and the phenolic and/or glycoalkaloid compounds are retained in a second retentate and wherein the addition of the one or more second salts and water of step (iii) is fully, or predominantly, performed using the one or more first salts and water present in the second permeate obtained in step (iv).

In another preferred embodiment, the second permeate obtained in step (iv) is subjected to a reverse osmosis membrane filtration step or evaporation step and the resulting water is used, at least in part, as the source of the water in step (iii). In other words, in a preferred embodiment the addition of water of step (iii) is fully, or predominantly, performed using the water separated from the second permeate obtained in step (iv) by a reverse osmosis membrane filtration step or evaporation step.

As will be appreciated by those skilled in the art, the diafiltration process with this recycle stream can be performed in many ways. When starting the diafiltration process, no second permeate is available yet. In a first embodiment, the diafiltration step (iii) starts with diafiltration liquid without added second salts and as soon as the second permeate is available from step (iv), this second permeate is recycled and used as diafiltration liquid in step (iii). In this first embodiment, the one or more second salts thus solely originate from the one or more first salts. In a second embodiment, the diafiltration step (iii) starts with diafiltration liquid with added second salts and as soon as the second permeate is available from step (iv), at least part this second permeate is recycled and used as diafiltration liquid in step (iii) or added to a stream of fresh water with added second salts. In this second embodiment, the one or more second salts thus originate from the one or more first salts and one or more second salts added to in step (iii). Continuously adding fresh diafiltration liquid in step (iii) and recycling second permeate to the diafiltration liquid may require purging part of the second permeate to avoid buildup of the diafiltration flow. Continuously adding fresh diafiltration liquid in step (iii) and recycling second permeate to the diafiltration liquid combined with purging part of the second permeate may be advantageous to avoid buildup of too high concentrations of salts in the diafiltration liquid.

For certain applications, it is preferred that the liquid comprising the separated potato proteins, *i.e.* the retentate obtained in diafiltration step (iii) using the first cross-flow membrane filtration, has a low level of turbidity and it may therefore be advantageous to perform a further separation step to remove suspended or colloidal particles from that liquid. The skilled person wanting to remove suspended or colloidal particles would consider removing them upstream in the process, preferably already from the potato fruit juice before step (i). Centrifugation is a very well-known method for separation of suspended solids from liquid solutions. However, potato fruit juice or derivatives thereof may be extremely difficult and/or expensive to clarify by centrifugation at large scale. Microfiltration is an alternative method for clarification but also this method has serious shortcomings due to fouling of the membrane surface with low productivity and low product permeability as the result. Therefore, it is not possible to efficiently apply microfiltration on crude potato fruit juice without serious loss of product and long processing times.

It has now surprisingly been found that the microfiltration process shows significantly less tendency to fouling when the liquid to be filtered has already been separated from phenolic and/or glycoalkaloid compounds that otherwise tend to adsorb to the surface of the membrane and block the pores.

Thus, in a preferred embodiment, the method as defined hereinbefore comprises the steps of:
(i) providing a potato fruit juice or a derivative thereof comprising proteins, one or more first salts and phenolic and/or glycoalkaloid compounds;
(ii) subjecting said potato fruit juice or the derivative thereof to a first cross-flow membrane filtration process wherein at least a portion of the one or more first salts and at least a portion of the phenolic and/or glycoalkaloid compounds migrate across the membrane into a first permeate and the potato proteins are retained in a first retentate;
(iii) subjecting the first retentate of step (ii) to a diafiltration step comprising adding one or more second salts and water to the first retentate while continuing the membrane filtration process to create a diafiltrate containing at least a portion of said phenolic and/or glycoalkaloid compounds and the added second salts and a retentate;
(iv) subjecting the first permeate and/or said diafiltrate from said first cross-flow membrane filtration process to a second cross-flow membrane filtration process wherein at least a portion of the salts present therein migrate across the membrane into a second permeate and the phenolic and/or glycoalkaloid compounds are retained in a second retentate; and
(v) subjecting the first retentate obtained in step (iii) to a microfiltration membrane filtration process wherein at least a portion of the potato proteins soluble in said first retentate migrate across the membrane into a microfiltration permeate with a lower turbidity than the turbidity of said first retentate.

In a preferred embodiment, the microfiltration permeate of step (v) is further treated with a third cross-flow membrane filtration process wherein the potato protein is concentrated in a third retentate and the salts are migrating through the membrane to create a third permeate.

In a further preferred embodiment, the third permeate is used as a diafiltration liquid to be added during the microfiltration process of step (v) to wash out further proteins from the retentate into the microfiltration permeate.

### Potato fruit juice or a derivative thereof

As explained hereinbefore, the term *'potato fruit juice or derivatives thereof'* means the liquid stream after the (industrial scale) separation of potato starch from potatoes (*solanum tuberosum* L.) and any aqueous streams derived from processing of such liquid stream by for example flocculation of contaminants and centrifugation, provided that the *'derivatives thereof'* still comprise protein, phenolic compounds and (first) salts.

The potato fruit juice or the derivative thereof that is used as a raw material in the process according to the present invention may be obtained by disintegration, e.g. by grinding, shredding and/or pressing, of the raw potatoes, whereby an aqueous solution comprising protein, salts, phenolic compounds and glycoalkaloid compounds is released as a juice, and/or the components may be extracted by addition of water or an aqueous extractant solution in combination with physical disruption of the plant tissue and/or cells. Antioxidants, such as sodium sulfite, sodium metabisulfite and ascorbic acid, may preferably be added prior to or during the procedures performed to produce the potato fruit juice or the derivative thereof. Likewise, it may be preferable to add salts to control conductivity and pH controlling substances such as acids or bases to adjust pH to a preferred value.

The potato fruit juice may be subjected to various pretreatment procedures creating a derivative of the potato fruit juice, such as deaeration, defoaming, solid-liquid separation (such as filtration, centrifugation, decantation, passing through hydroclyclones), heating or cooling.

The potato fruit juice or a derivative thereof so obtained may be pretreated in various ways prior to separation according to the invention. Preferably the juice is pretreated to remove insoluble material of a particle size larger than 10 micron which may be achieved *e.g.* by centrifugation and/or filtration.

In a preferred embodiment the conductivity of the potato fruit juice or the derivative thereof is, or is adjusted to, in the range of 1 to 50 mS/cm, such as 2 to 40 mS/cm, 3 to 30 mS/cm, 3 to 25 mS/cm, 3 to 20 mS/cm, or 5 to 17 mS/cm.

In a preferred embodiment the pH of the potato fruit juice or the derivative thereof is within the range of 4.5 to 8.5, such as 5.2 to 7.5. For certain applications, the pH is preferably in the range of 4.5 to 6.5, such as 4.8 to 6.5, or 5.0 to 6.5. In a preferred embodiment, the pH of the potato fruit juice or the derivative thereof is adjusted to a pH in the range 4.5 to 8.5, such as in the range of 5.2 to 7.5, preferably in the range of 4.5 to 6.5, such as in the range of 4.8 to 6.5, or 5.0 to 6.5 prior to said first cross-flow membrane filtration process.

### Potato proteins

In an embodiment, the potato proteins to be separated are selected from the group consisting of patatin, protease inhibitors, lipoxygenase, polyphenol oxidase, acid and alkaline phosphatase, or mixtures thereof, preferably patatin or protease inhibitors, or mixtures thereof.

In an embodiment, the potato protein to be separated is patatin. In another embodiment, the potato protein to be separated is protease inhibitors.

### Phenolic and/or glycoalkaloid compounds

As explained hereinbefore, the potato fruit juice or the derivative thereof contains phenolic and/or glycoalkaloid compounds, such as (a) phenolic acids, flavonoids, tannins, stilbenes, lignans, gallic acid and/or catechin and (b) α-solanine and/or α-chaconine.

In an embodiment, the phenolic compounds to be separated are selected from the group consisting of phenolic acids and flavonoids including flavonols, flavanols, and anthocyanins. In another embodiment, the phenolic compounds to be separated are selected from the group consisting of phenolic acids and flavonoids including flavonols, flavanols, and anthocyanins, and combinations thereof.

In an embodiment, the phenolic compounds to be separated comprise chlorogenic acids. In another embodiment, the phenolic compounds to be separated comprise catechin.

In an embodiment, the glycoalkaloid compounds to be separated comprise α-solanine and/or α-chaconine.

In an embodiment, the phenolic and glycoalkaloid compounds to be separated are selected from the group consisting of (a) phenolic acids and flavonoids, including flavonols, flavanols, and anthocyanins, and (b) α-solanine and α-chaconine, and combinations thereof.

### Fibers

The potato fruit juice or the derivatives thereof will most often contain insoluble fibers, also known as dietary fibers. Such dietary fibers may have health promoting effects and add functionality when applied as a food ingredient.

In a preferred embodiment, the potato fruit juice or the derivative thereof comprising potato proteins, one or more first salts and phenolic and/or glycoalkaloid compounds also contains insoluble fibers at a concentration in the range of 0.1 to 30 mg/ml, based on the dry weight of the insoluble fiber, such as 0.2 to 20 mg/ml, 0.3 to 15 mg/ml, 0.5 to 10 mg/ml, or 1.0 to 5 mg/ml, based on the dry weight of the insoluble fiber.

In an embodiment, more than 80 % of said insoluble fibers in the potato fruit juice are small enough to pass a 50 micron filter, such as a 40 micron filter, a 30 micro filter, a 20 micron filter, a 10 micro filter, a 5 micron filter, or a 2 micron filter.

In an embodiment, said insoluble fibers are present in the potato fruit juice in a concentration that results in a pellet when the fruit juice is centrifuged in a tabletop centrifuge at 4000 G for 30 min.

In an embodiment, said pellet constitutes in the range of 0.1 to 10 vol/vol %, such as 0.1 to 5 vol/vol %, 0.1 to 3 vol/vol %, 0.1 to 2 vol/vol %, 0.1 to 1 vol/vol %, 0.2 to 5 vol/vol %, 0.2 to 3 vol/vol %, 0.3 to 5 vol/vol %, 0.3 to 3 vol/vol %, 0.4 to 5 vol/vol %, 0.4 to 4 vol/vol %, 0.5 to 5 vol/vol %, 0.5 to 4 vol/vol %, or 0.5 to 3 vol/vol %, when the potato fruit juice at a pH of 6 to 7 has been diluted (or concentrated) to a true protein concentration of 5 mg/ml.

### First cross-flow membrane process

In a preferred embodiment, the first cross-flow membrane filtration process to provide the first permeate and the first retentate according to step (ii) of the process as defined hereinbefore is an ultrafiltration process wherein high molecular weight compounds, such as proteins, are retained in the first retentate and low molecular weight substances, such as salts and phenolic and/or glycoalkaloid compounds migrate across the membrane as a first permeate.

Preferably the pore size of the ultrafiltration membrane is in the range of 5 to 500 kDa, such as 10 to 500 kDa, 10 to 200 kDa, 10 to 100 kDa, 10 to 50 kDa, 30 to 500 kDa, or 50 to 500 kDa.

More preferably, the pore size of the ultrafiltration membrane is in the range of 10 to 50 kDa, such as about 30 kDa.

The membrane material may be any kind of membrane suitable for cross-flow filtration, examples of which are polyamide, polysulfone, poyethersulfone, polyvinyldifluoride, polyacrylonitrile, cellulose acetate, permanently hydrophilized polysulfone, permanently hydrophilized polyethersulfone and ceramic membranes. Preferably, the membrane material is regenerated cellulose, polypropylene or polyethylene. Most preferably, the membrane material is selected from the group consisting of polysulfone, poyethersulfone permanently hydrophilized polysulfone and permanently hydrophilized polyethersulfone.

In an embodiment, the membrane is a flat sheet or a spiral wound flat sheet membrane.

In an embodiment, the membrane is a hollow fiber or tubular membrane. In a preferred embodiment, the hollow fiber membrane has an internal diameter in the range of 0.5 to 3.0 mm, such as in the range of 0.6 to 2.5 mm, 0.7 to 2.2 mm, 0.8 to 2.0 mm, 0.9 to 1.8 mm, 1.0 to 1.7 mm, or 1.1 to 1.6 mm.

In a preferred embodiment, the hollow fiber membrane has a length in the range of 30 to 150 cm, such as in the range of 40 to 140 cm, 50 to 130 cm, 60 to 120 cm, 70 to 115 cm, 80 to 110 cm, or 90 to 110 cm.

In a preferred embodiment, the hollow fiber membrane material comprises polysulfone, cellulose acetate, ceramic material, polyamide, polyethersulfone or permanently hydrophilized polysulfone or polyethersulfone.

In a preferred embodiment, the hollow fiber membrane has a pore size in the range of 5 kDa to 500 kDa, such as in the range of 10 kDa to 200 kDa, in the range of 20 kDa to 100 kDa, or in the range of 30 kDa to 75 kDa.

In a preferred embodiment, the hollow fiber membrane is an inside-out membrane wherein the transmembrane transport of liquid is performed from the inner lumen of the membrane to the outside surface of the membrane.

### Second cross-flow membrane filtration process

In a preferred embodiment, the second cross-flow membrane filtration process to provide the second permeate and the second retentate according to step (iv) of the process as defined hereinbefore is an nanofiltration process wherein phenolic and/or glycoalkaloid compounds are retained in the second retentate and first and second salts migrate across the membrane as a second permeate.

Preferred materials for the nanofiltration membrane include organic polymers, such as cellulose acetate, polysulfone, poly(ether)sulfone, sulfonated polysulfone, aromatic polyamides, polyimide, poly(piperazine amide) and blends and composites thereof.

The pore size of the nanofiltration membrane is preferably in the range of 100-1000 Da, such as 200-800 Da, or 300-700 Da. For some applications, the pore size is preferably in the range of 500-800 Da, such as 300-500 D, or 150-300 Da.

Especially interesting nanofiltration devices for the separation of polyphenols and/or glycoalkaloids are Filmtec NF elements from Dupont, USA, Duracon and Dairy Thin film NF elements from SUEZ Water Technologies & Solutions, USA, NFX, NFW and NFG elements from Synder Filtration, USA, vNF Spiral thin film composite membranes from Alfa Laval Corp, Sweden, and X-Flow hollow fiber nanofiltration elements from Pentair, The Netherlands.

### Diafiltration step

In order to optimize the processing logistics, and for some applications the processability of said first retentate, the first retentate obtained in step (ii) may advantageously be stored for several hours before the diafiltration of step (iii) is carried out. Thus, in an embodiment, said first retentate is stored for at least 3 hours, such as at least 6 hours, at least 12 hours, at least 18 hours, at least 24 hours, at least 36 hours, or at least 48 hours before the diafiltration step of (iii) is performed. In a further embodiment, the diafiltration step (iii) may be partly carried out before the storage of said first retentate.

In yet a further embodiment, said first retentate obtained in step (ii) may be treated with a preliminary diafiltration step using e.g. water or a low conductivity aqueous solution prior to storage and subsequent diafiltration according to step (iii).

In an embodiment, the storage temperature of said first retentate is within the range of 1 to 25 °C, such as 2 to 20 °C, 3 to 15 °C, 2 to 12 °C, 2 to 10 °C, or 2 to 8 °C.

In a further embodiment, the pH of said first retentate during storage is, or is adjusted to, a pH in the range of 4.5 to 5.6, such as 4.5 to 5.4, 4.5 to 5.2, or 4.7 to 5.3.

As will be appreciated by those skilled in the art, the wording *'while continuing the membrane filtration process'* in step (iii) means that the first cross-flow membrane filtration process in step (ii) and the diafiltration step (iii) are performed across the same membrane, the difference being that step (ii) is used without diafiltration liquid resulting in concentration, whereas step (iii) is performed with diafiltration liquid (the one or more second salts and water).

The one or more second salts to be added in step (iii) of the method as defined herein may be any water soluble salt of on the one hand one or more of sodium, potassium, ammonium, calcium and magnesium and on the other hand one or more of mineral acids and organic acids.

In a preferred embodiment, the one or more second salts are selected from sodium, potassium, ammonium, calcium or magnesium salts of a mineral acid or an organic acid.

Examples of suitable organic acids are formic acid, acetic acid, propanoic acid, citric acid, caprylic acid, lactic acid, gluconic acid, tartaric acid, fumaric acid, butanedioic acid and benzoic acid.

As explained hereinbefore, the membrane applied in the second cross-flow membrane filtration step (iv) preferably is a nanofiltration membrane. In order to achieve the highest permeability of the salt through a nanofiltration membrane retaining the phenolic compounds in the retentate, the one or more second salts are preferably chosen from the group of salts constituted by only monovalent inorganic anions and cations, such as sodium chloride, potassium chloride and ammonium chloride.

However, in certain applications there will be a need to limit process costs and the cost of environmentally friendly waste handling. Likewise, certain factory designs cannot withstand the corrosive nature of *e.g.* chlorides. For such applications, the one or more salts are preferably chosen from the group of sodium sulfate and potassium sulfate.

During diafiltration step (iii), the one or more second salts may be added to the retentate as an aqueous solution or as a solid to be solubilized in the first retentate.

The amount of one or more second salts added determines the conductivity of the retentate during diafiltration step (iii).

In an embodiment, the electrical conductivity of the retentate during the diafiltration step (iii), preferably during step (ii) and diafiltration step (iii), remains within the range of 1 to 50 mS/cm, such as in the range of 2 to 40 mS/cm, 3 to 30 mS/cm, 3 to 25 mS/cm, 3 to 20 mS/cm, 5 to 17 mS/cm, 6 to 25 mS/cm, 7 to 25 mS/cm, or 10 to 25 mS/cm.

More preferably, the electrical conductivity of the retentate during the diafiltration step (iii), preferably during step (ii) and diafiltration step (iii), remains within the range of 3 to 20 mS/cm.

The nature of the one or more second salts added during diafiltration step (iii) may also influence the pH of the retentate. In a preferred embodiment the pH of the retentate during diafiltration step (iii) remains within the range of 4.5 to 8.5, such as 5.2 to 7.5.

In a particular embodiment, the diafiltration in step (iii) is performed first at a relatively low temperature to remove the majority of the phenolic and/or glycoalkaloid compounds followed by diafiltration at a relatively higher temperature to remove the remaining phenolic and/or glycoalkaloid compounds. Thus, in a preferred embodiment the retentate in a first phase of diafiltration remains at a temperature in the range of 1 to 30 °C, such as 5 to 28 °C, 10 to 25 °C, or 15 to 22 °C, followed by a second phase of diafiltration wherein the retentate remains at a temperature in the range of 30 to 60 °C, such as 30 to 55 °C, 35 to 52 °C, 38 to 50 °C, or 40 to 48 °C.

For certain applications, the pH of the retentate during diafiltration step (iii) preferably remains in the range of 4.5 to 6.5, such as 4.8 to 6.5, or 5.0 to 6.5.

In a preferred embodiment, the retentate during diafiltration step (iii) remains at a temperature in the range of 1 to 60 °C, such as 5 to 55 °C, 10 to 50 °C, 15 to 48 °C, 12 to 45 °C, 18 to 30 °C, or 22 to 28 °C.

The addition of the one or more second salts and water in diafiltration step (iii) is to be performed until enough volume has been added, and subsequently removed via the permeate, to reach a satisfactory (low) level of phenolic and/or glycoalkaloid compounds in the retentate.

In an embodiment, the target value for remaining phenolic and/or total glycoalkaloid compounds in the retentate of diafiltration step (iii) corresponds to less than 5000 mg phenolic and/or total glycoalkaloid compounds per kg potato protein on the basis of dry weight, such as less than 4000 mg/kg, less than 3000 mg/kg, less than 2000 mg/kg, less than 1500 mg/kg, less than 1250 mg/kg, less than 1000 mg/kg, less than 750 mg/kg, less than 500 mg/kg, less than 200 mg phenolic and/or total glycoalkaloid compounds/kg potato protein on the basis of dry weight.

In some applications it is further important to reach a lower level of phenolic and/or total glycoalkaloid compounds having a molecular weight below 10 kDa. Thus, in a further embodiment, the target value for phenolic and/or total glycoalkaloid compounds having a molecular weight below 10 kDa remaining in the retentate of diafiltration step (iii) corresponds to less than 1500 mg/kg, such as less than 1000 mg/kg, less than 750 mg/kg, less than 500 mg/kg, less than 300 mg/kg, less than 200 mg/kg, less than 100 mg/kg, or less than 50 mg phenolic and/or total glycoalkaloid compounds/kg protein on the basis of dry weight.

In an embodiment, glycoalkaloids are efficiently separated from the potato proteins present in the potato fruit juice or the derivative thereof. Thus, in an embodiment, diafiltration step (iii) brings the total glycoalkaloids remaining in the retentate below a target amount corresponding to less than 500 mg/kg, such as less than 300 mg/kg, less than 200 mg/kg, less than 100 mg/kg, or less than 50 mg total glycoalkaloids/kg potato protein on the basis of dry weight.

In an embodiment, the addition of one or more second salts and water in diafiltration step (iii) is performed continuously and with practically the same flow rate as the diafiltrate is migrating through the membrane. In another embodiment, the addition of one or more second salts and water in diafiltration step (iii) is done in small portions, such as portions approximately equal to the retentate volume.

In an embodiment, the total volume of water added during diafiltration step (iii) is in the range of 4 to 20 times the volume of the first retentate, such as 4 to 15 times, 5 to 12 times, 5 to 10 times, 6 to 10 times, 7 to 9 times, or 5 to 9 times the volume of the first retentate. More preferably, the total volume of water added during diafiltration step (iii) is in the range of 6 to 10 times the volume of the retentate.

In a preferred embodiment, diafiltration step (iii) comprises a second phase of diafiltration following the addition of one or more second salts and water, wherein the diafiltration is continued with the addition of water without adding any further salts. In this way the salt concentration and conductivity of the retentate will gradually decrease until a target value for the conductivity has been reached.

In an embodiment, the second phase of diafiltration in step (iii) is continued with water and without the addition of further salts until the conductivity of the retentate is less than 10 mS/cm, such as less than 8 mS/cm, less than 5 mS/cm, less than 4 mS/cm, less than 3 mS/cm, less than 2 mS/cm, or less than 1 mS/cm.

In order to optimize the processing logistics, and for some applications the processability of the retentate resulting after diafiltration step (iii), the retentate may advantageously be stored for several hours before further processing, such as a preferred microfiltration step (v), drying or any further separation steps are carried out.

Thus, in an embodiment, the retentate resulting after the diafiltration step (iii) is stored for at least 3 hours, such as at least 6 hours, at least 12 hours, at least 18 hours, at least 24 hours, at least 36 hours, or at least 48 hours before further processing is performed.

In an embodiment, the storage temperature of said retentate resulting after diafiltration step (iii) is within the range of 1 to 25 °C, such as 2 to 20 °C, 3 to 15 °C, 2 to 12 °C, 2 to 10 °C, or 2 to 8 °C.

In a further aspect the pH of said retentate resulting after diafiltration step (iii) during storage is, or is adjusted to, a pH in the range of 4.5 to 5.6, such as 4.5 to 5.4, 4.5 to 5.2, or 4.7 to 5.3.

### Inactivation of unwanted enzymatic activities

In a preferred embodiment, the first retentate obtained in step (ii) or the potato proteins isolated according to the invention are treated to provide inactivation of enzymatic activities that may be unwanted when the potato proteins are to be used as food ingredients in certain applications. In particular, inactivation of the esterase and lipolytic activity of patatins and the inactivation of polyphenol oxidase activity is preferred. It is well known that such inactivation may be performed by extensive heating of the proteins. However, such treatment may also lead to uncontrolled polymerization and gelling/denaturation of the proteins, such that the functionality for use in foods is lost or at least significantly decreased.

The inventors have surprisingly found that treatment of the first retentate obtained in step (ii) or the potato proteins isolated according to the invention with acidic pH values for a limited period of time may lead to the preferred inactivation, particularly inactivation of the esterase and lipolytic activity of patatins, in a controlled manner without losing the functionality of the potato proteins. Moreover, the inventors have surprisingly found that this inactivation, particularly inactivation of the esterase and lipolytic activity of patatins, improves the flavor of the potato proteins isolated according to the invention.

The wording *'or the potato proteins isolated according to the invention'* refers to any one of the following:
- the retentate resulting from diafiltration step (iii);
- the microfiltration permeate obtained in step (v);
- the third retentate obtained by treating the microfiltration permeate of step (v) with a third cross-flow membrane filtration process; or
- any other aqueous stream comprising potato proteins obtained downstream of diafiltration step (iii).

Thus, in a preferred embodiment, the first retentate obtained in step (ii) or a solution of the potato proteins isolated according to the invention, preferably the retentate resulting from diafiltration step (iii), is exposed to pH values between 2.0 and 4.5, such as between 2.0 and 4.0, between 2.0 and 3.75, between 2.0 and 3.5, between 2.0 and 3.2, or between 2.0 and 3.0 in a time and temperature interval sufficient to eliminate unwanted enzymatic activity of the potato proteins without adversely affecting functionality of the potato proteins.

As will be appreciated by those skilled in the art, the wording *'without adversely affecting functionality of the potato proteins'* is to be understood as *'without substantially adversely affecting functionality of the potato proteins'* because subjecting the potato proteins to the acidic pH values for a limited period of time may negatively affect at least part of the functionality of the potato proteins.

In an embodiment, the first retentate obtained in step (ii) or the potato protein solution isolated according to the invention, preferably the retentate resulting from diafiltration step (iii), exposed to the low pH comprises patatin and the enzymatic activity to be eliminated is the esterase activity of said patatin.

In an embodiment, the first retentate obtained in step (ii) or the potato protein solution isolated according to the invention, preferably the retentate resulting from diafiltration step (iii), exposed to the low pH comprises one or more polyphenol oxidases and the enzymatic activity to be eliminated is the oxidative activity of said polyphenol oxidase.

In an embodiment, the first retentate obtained in step (ii) or the potato protein solution isolated according to the invention, preferably the retentate resulting from diafiltration step (iii), exposed to the low pH comprise phospholipase and the enzymatic activity to be eliminated is the phospholipase activity of said phospholipase.

In an embodiment, the inactivation of enzymatic activity is performed at a temperature in the range of 1-70 °C, such as in the range of 1-65 °C, 5-60 °C, 5-15 °C, 10-55 °C, 10-15 °C, 15-55 °C, 18-50 °C, 18-48 °C, or 20-45 °C.

In an embodiment, the inactivation of enzymatic activity is performed in a time span of at least 15 second, such as at least 5 minutes, such as at least 10 minutes, such as at least 30 minutes, such as at least 60 minutes, such as at least 120 minutes, such as at least 240 minutes.

In a very preferred embodiment, the inactivation of enzymatic activity is performed by exposing the first retentate obtained in step (ii) or a solution of the potato proteins isolated according to the invention, preferably the retentate resulting from diafiltration step (iii), to:
- a pH value between 2.0 and 4.5, preferably between 2.0 and 4.0, more preferably between 2.0 and 3.75, even more preferably between 2.0 and 3.5, such as between 2.0 and 3.2, or between 2.0 and 3.0;
- a temperature in the range of 1-70 °C, preferably in the range of 5-60 °C, more preferably in the range of 10-55 °C, even more preferably in the range of 20-45 °C; and
- in a time span of up to 240 minutes, preferably between 15 seconds and 120 minutes, more preferably between 5 and 60 minutes, such as about 15, 20, 30 or 45 minutes.

In a preferred embodiment, the pH of the thus treated stream is brought back to a value between 4.5 and 8.5, such as between 6 and 8, or between 6.5 and 7.5. As will be appreciated by those skilled in the art, such an additional step is not needed if the step of inactivation of enzymatic activity is performed just before a diafiltration step wherein diafiltration liquid is added, automatically resulting in an increase of the pH.

The inactivation of enzymatic activity is preferably performed by exposing the first retentate obtained in step (ii) before diafiltration step (iii) to the low pH. Before diafiltration in step (iii) and after the concentration in step (ii), the volumetric flow rate is reduced, such that the amount of acid needed to reduce the pH is limited. Moreover, by subjecting thus treated retentate to diafiltration in step (iii), the pH can be quickly brought back to a value of 4.5 or higher to avoid overexposure of the potato proteins.

More preferably, the inactivation of enzymatic activity is performed by exposing the retentate resulting from diafiltration step (iii) to the low pH. Since the polyphenols have been sufficiently removed from this stream, the risk of unwanted chemical reactions at acidic pH is eliminated.

It was further surprisingly found that a controlled and limited proteolysis of the potato proteins isolated according to the invention may lead to inactivation of unwanted enzymatic activity without destroying important functional properties, such as the ability to form food gels.

Thus, in an embodiment, the inactivation of unwanted enzymatic activity of the potato protein isolated according to the invention is achieved by treatment of a solution of the potato protein with one or more proteolytic enzymes. In an embodiment, the enzymatic activity of patatin is in part or fully inactivated by the treatment with a protease. In an embodiment, the enzymatic activity of phospholipase is in part or fully inactivated by the treatment with a protease. In an embodiment, the enzymatic activity of polyphenol oxidase is in part or fully inactivated by the treatment with a protease.

In an embodiment, the one or more proteases are endopeptidase. In an embodiment, the one or more proteases are serine proteases. In an embodiment, the one or more proteases are chosen from the group of trypsin-like, chymotrypsin-like, thrombin-like, elastase-like and subtilisin-like proteases. In an embodiment, the one or more proteases are subtilisin.

### Inactivation of microbial growth during processing

Potato fruit juice and other extracts of potato proteins may contain a high level of microbes naturally originating from the growth and storage of the potatoes in the soil. It is therefore preferred to add a germicidal step during the process according to the invention. This step may be applied directly to the crude potato fruit juice, or it may preferably be applied during and/or in between the membrane filtration processing steps according to the invention.

Thus, in an embodiment, the germicidal step is performed on the potato fruit juice or the derivative thereof provided in step (i) prior to step (ii). Before diafiltration in step (iii) and after the concentration in step (ii), the volumetric flow rate is reduced. Accordingly, it may be advantageous to perform the germicidal step just before step (iii). Thus, in a preferred embodiment, the germicidal step is performed on the first retentate provided in step (ii) prior to diafiltration step (iii).

Since many proteins are highly sensitive to classical pasteurization by heat treatment, leading to loss of functional properties of the proteins, the germicidal step is performed at a temperature in the range of 2-60 °C, such as in the range of 5-55 °C, in the range of 8-52 °C, in the range of 10-50 °C, in the range of 12-45 °C, in the range of 12-25 °C, or in the range of 12-18 °C.

The germicidal step is applied using high intensity UV light, X-rays, pulsed electric field treatment or high-pressure pasteurization.

In an embodiment, the germicidal step comprises passing of the potato fruit juice or the derivative thereof through a photo bioreactor illuminating high intensity UV light to the fruit juice. In an embodiment, the UV light has a wavelength primarily in the range of 180-300 nm. In an embodiment, the potato fruit juice or the derivative thereof is illuminated with UV light when passing one or more a spiral-shaped tubes, allowing the passage of the UV light. In an embodiment, the inner diameter of said spiral tubes is in the range of 1-15 mm, such as in the range of 2-12 mm, such as in the range of 3-10 mm, such as in the range of 4-9 mm. In an embodiment, the potato fruit juice or the derivative thereof is passed more than one time through the photo bioreactor.

In an embodiment, the potato fruit juice or the derivative thereof is passing the photo bioreactor simultaneously with one or more of the membrane filtration steps according to the invention. In an embodiment, the photo bioreactor is inserted into the recirculation loop of one or more of the membrane filtration units applied according to the invention such that the potato fruit juice or the derivative thereof is continuously passing the photo bioreactor. In an embodiment, the photo bioreactor is inserted as a shunt to the recirculation loop of one or more of the membrane filtration units applied according to the invention. In an embodiment, the photo bioreactor is inserted as a recirculation unit to the holding tank of one or more of the membrane filtration units applied according to the invention. In an embodiment, the germicidal step using a photo bioreactor is applied to the potato protein solution after the membrane filtration steps according to the invention. In an embodiment, the O.D.600 nm absorption of the resulting protein solution is in the range of 0.02 to 15.0 , such as in the range of 0.05 to 8.0, in the range of 0.1 to 7.0, in the range of 0.5 to 6.0, in the range of 0.8 to 5.0, in the range of 0.9 to 4.0, or in the range of 1.0 to 3.0.

In an embodiment of the invention the photo bioreactor is substantially equal to the photo bioreactor disclosed in WO2019/057257A1, which is hereby incorporated by reference.

### EXAMPLES

### Materials

Chemicals used in the examples herein *e.g.* for preparing buffers and solutions are commercial products of at least reagent grade. Water used for conducting the experiments is all de-ionized water.

### Potato fruit juice and derivatives thereof

Potatoes of the variety Folva were obtained from a local supermarket. The potatoes were washed and their surfaces were dried off before the potatoes were shredded including the peel, while the liberated liquid (juice) concomitantly was separated from the main mass of insolubles using a commercial juicer (Nutrijuicer PRO) without diluting with water. A volume of 10 ml sodium sulphite (10 wt%) per 1000 ml juice was added immediately to the juice and the juice was centrifuged for 5 min at 1430 G to remove any remaining large insoluble particles such as insoluble fibers and starch.

An amount of 10 kg of potatoes yielded about 4.65 L of centrifuged juice with a pH of 6.2 and a conductivity of 10.7 mS/cm, as measured with a Seven2Go S3 conductivity meter from Mettler Toledo, Switzerland. The so obtained potato fruit juice did not contain visible particles larger than about 10 µm, but nevertheless remained unclear with suspended colloid particles.

### Buffer solutions

(I) A 10 wt% sodium sulphite buffer solution was prepared by dissolving 10 g of sodium sulphite from Sigma Aldrich USA (cat. No.: 13471) in water to a total volume of 100 ml;
(II) A 0.1 M di-potassium hydrogen phosphate pH 7.0 buffer solution was prepared by dissolving 17.42 g dipotassium hydrogen phosphate from Sigma Aldrich USA (cat.no.: P3786) in 900 ml water followed by adjusting pH to 7.0 with 4 M hydrochloric acid. Finally, water was added to obtain a final volume of 1 L;
(III) A 0.1 M sodium sulfate solution was prepared by dissolving 14.2 g anhydrous sodium sulfate SL 99.8 % from Lenzing, Austria, in water to a total volume of 1000 ml;
(IV) A 0.1 M pyrocatechol in 0.1 M di-potassium hydrogen phosphate pH 7.0 buffer solution (for PPO detection) was prepared by dissolving 11.01 g pyrocatechol from Sigma Aldrich, USA (cat.no.: C9510) in 100 ml 0.1 M di-potassium hydrogen phosphate pH 7.0;
(V) 20 mg/ml 1-naphtyl acetate in 2-propanol: 80 mg 1-naphtyl acetate from Sigma, USA (Cat.no.: N8505), 4 ml of 2-propanol from VWR BDH Chemicals (Cat.no.: 20922.364);
(VI) 0.1 M tris pH 7.5: 12.1 g Tris VWR Life Science (Cat.no.: 0826), add 900 ml of water adjust pH to 7.5 with 4 % HCl, add water to 1 L;
(VII) Fast Blue BB solution 6 mg/ml in 10 % SDS (freshly made, kept in the dark): 30 mg of Fast Blue BB Salt hemi(zinc chloride) from Sigma, USA (Cat.no.: F3378), 5 ml 10 % SDS;
(VIII) 0.2 mg/ml 1-naphtyl acetate in 0.1 M tris pH 7.5 (freshly made): 30 ml 0.1 M tris pH 7.5, 0.3 ml 20 mg/ml 1-naphtyl acetate in 2-propanol.

### SDS-PAGE electrophoresis reagents

LDS sample buffer, 4X was obtained from Expedeon, USA (Cat.no.: NXB31010)
SDS Run buffer, 20x was obtained from Expedeon, USA (Cat.no.: NXB50500)
Precast 4-20% gradient gels were obtained from Expedeon, USA (Cat.no.: NXG42012K)
Instant Blue Coomassie staining solution was obtained from Expedeon, USA (Cat.no.ISB1L).

### Assays

### Esterase activity

To 3 ml 0.2 mg/ml 1-naphtyl acetate in 0.1 M tris pH 7.5 (fresh made), 0.3 ml potato protein sample is added. The solution is mixed well and incubated for exactly 15 minutes. 0.42 ml of Fast Blue BB solution 6 mg/ml in 10 % SDS is added. The resulting solution is mixed well and incubated for exactly 10 minutes.

A reference sample of 0.3 ml tris buffer is made up (= blind). The samples are analysed at 510 nm (O.D.510) using a spectrophotometer (BK-UV1800 spectrophotometer, Biobase, China). The 510 nm reading from the blind is subtracted from the 510 nm reading from the potato protein sample. The result is expressed as *'esterase activity signal'* and is used as a relative measure to compare different samples within the same experiment (*i.e.* without transforming into a units/mg expression of activity).

### SDS-PAGE electrophoresis

The samples produced in the examples were analyzed using SDS-PAGE gel electrophoresis to determine the protein composition in each sample. The SDS-PAGE gel electrophoresis was performed using an electrophoresis apparatus and precast 4-20% gradient gels from Expedeon USA (Cat.no.: NXG42012K). The protein samples were mixed with LDS sample buffer and incubated for 10 minutes at 70 °C. The samples were applied to a precast gel and the proteins were allowed to run for one hour at 200 V 90 mA in the SDS Run buffer at non-reduced running conditions. The gel was developed in the staining solution for three hours and the protein bands were evaluated by visual inspection or analyzed by scanning densitometry to quantify the amount of specific proteins in the test solutions.

### Dry matter determination

A Sartorius moisture analyzer (MA37, Sartorius) was used to determine dry matter in a sample by applying 5-10 mL of a sample to the instrument. The sample was dried at 110 °C until constant weight and the remaining dry matter was determined and calculated by the instrument.

### Semi-quantitative determination of polyphenol oxidase (PPO) activity

A sample was mixed with a pyrocathecol solution pH 7.0 at ambient temperature. If the sample contained PPO, the solution changed color from colorless to orange within 15 minutes. The color intensity obtained after 15-minute incubation at room temperature indicates the amount of PPO enzyme activity in the sample. A sample with an unknown amount of enzyme activity may quickly be estimated relative to the activity expressed by a reference sample (*e.g.* the starting material) and dilutions hereof by visual inspection.

The procedure was as follows. A solution is prepared by combining 1.95 ml 0.1 M potassium phosphate pH 7.0, 1 ml 0.1 M pyrocatechol in 0.1 M potassium phosphate pH 7.0 and 50 µl of a sample. The resulting solution was mixed well and incubated for 15 minutes at ambient temperature. After 15 minutes, the color intensity was scored relative to a reference sample.

### Determination of total glycoalkaloids

A HPLC method for determination of total glycoalkaloids was applied according to V. Alt et al., Optimization of glycoalkaloid analysis for use in industrial potato fruit juice downstreaming, Eng. Life Sci., 2005, 5, pp 562-567. α-solanine (Sigma Aldrich, USA, cat no.: S3757) was used as a reference.

### Test for alkaline colored phenolic compounds

A semi-quantitative test for the content of complex phenolic compounds based on the development of color in alkaline medium was used. A sample with a volume of 0.5 ml was mixed with 3 ml 1 M sodium hydroxide. The color intensity of a set of samples was inspected visually and assigned a relative score as follows:
++++ means very strong color intensity, +++ means strong color intensity, ++ means clearly visible color intensity, + means weak color intensity, (?) means hardly visible color, ND means no visible color.

### True protein determination

True protein concentration was also determined by the method of Kjeldahl using the conversion factor N x 6,25. All samples were initially dialyzed against demineralised water in dialysis tubing cellulose membrane (Sigma-Aldrich, USA, cat. No.: D9652) to remove any free amino acids and low molecular weight peptides.

### Membrane filtration

Membrane filtration test runs were performed using a multipurpose pilot membrane filtration unit (PAN 4010 from Koch Membrane Systems, USA) equipped with the relevant membrane types as indicated in the examples.

### Example 1: Ultrafiltration and diafiltration of potato fruit juice

In examples 1A and 1B, two separate preparations of 30 L potato fruit juice, produced as described in sections *'Materials'* and *'Assays',* were subjected to ultrafiltration without any pretreatment followed by diafiltration using either water (Example 1A) or 0.1 M sodium sulfate (Example 1B) as the diafiltration liquid.

The procedure was as follows. For both tests a 3 inch ROMICON PM30, 1.1 mm i.d. (Koch Membrane Systems, USA) hollow fiber membrane unit with a cut off of 30 kDa was employed. This unit has a membrane area of 2.3 m². The cross flow was set at 34 L/min and the TMP (transmembrane pressure) was regulated to be approximately 1.2 bar. The two 30 L potato fruit juice preparations were each concentrated using ultrafiltration to a retentate volume of 6 L (*i.e.* the concentration factor was 5X) and the clear yellow/brown permeates were collected. Using the built-in heat exchanger of the equipment the temperature of the retentate was kept in the range of 17-20 °C during the entire ultrafiltration procedure.

Following the 5X concentration, the two preparations were diafiltered as follows.
1A) To the retentate was added 6 L water followed by further ultrafiltration to again reduce the retentate volume to 6 L while the corresponding permeate was collected in a separate vessel labelled *'dia-permeate 1A'.* This procedure was repeated to create in total 10 water dia-permeates each of 6 L volume. Following the 10^{th} collection of permeate with water one portion of 6 L 0.2 M sodium sulfate was added to the retentate and the ultrafiltration was continued to create the 11^{th} permeate. The permeate flux was measured during each of the 11 diafiltration steps and dia-permeate samples were subjected to a semi-quantitative test for the presence of alkaline colored phenolic compounds as described above;
1B) To the retentate was added 6 L 0.1 M sodium sulfate followed by further ultrafiltration to again reduce the retentate volume to 6 L while the corresponding permeate was collected in a separate vessel labelled *'dia-permeate 1B'.* This procedure was repeated to create in total 10 sodium sulfate dia-permeates each of 6 L volume. Following the 10^{th} collection of sodium sulfate permeate, three portions of 6 L water was added to the retentate and the ultrafiltration was continued to create the 11^{th}-13^{th} permeate. The permeate flux was measured during each of the 13 diafiltration steps and dia-permeate samples were subjected to a semi-quantitative test for the presence of alkaline colored phenolic compounds as described above.

The measured average flux values obtained from each dia-permeate is listed in Table 1. As can be seen in Table 1, the flux rates in Example 1A (using water for diafiltration) quickly declines from an initial level at 16 LMH (liter/m²/h) to 3 LMH with an average for the first 10 dia-permeates of 6.6 LMH. In contrast, the flux rates in example 1B (using 0.1 M sodium sulfate for diafiltration) show an increase from an initial level of 17 LMH to 20 LMH with an average for the first 10 dia-permeates of 18.5 LMH. The productivity of the membrane system employed was thus approx. 2.8 times higher when applying 0.1 M sodium sulfate for diafiltration relative to the use of water.

The semi-quantitative tests for alkaline colored phenols in the dia-permeates further shows that for both Examples 1A and 1B there is a strongly positive score in the first permeates while the scores for Example 1A dia-permeates gradually become lower than for the Example 1B dia-permeates indicating a gradually declining relative permeability of the phenolic substances when using water for diafiltration relative to 0.1 M sodium sulfate.

Moreover when changing from water to 0.2 M sodium sulfate for creation of dia-permeate 11A a significant increase in the score is observed, which further indicates that the addition of salts promotes the permeability of the phenolic compounds.

The flux decline from 20 LMH to 8 LMH in dia-permeates 11B to 13B illustrates that even when shifting to water for diafiltration after thorough diafiltration with sodium sulfate the flux is still dependent on added salts being present in the retentate.

**Table 1**

| **Dia-permeate** | **Flux, LMH** | **Alkaline phenol test** |
|---|---|---|
| 1A / 1B | 16 / 17 | ++++ / ++++ |
| 2A / 2B | 13 / 17 | ++++ / ++++ |
| 3A/3B | 9/17 | +++ / ++++ |
| 4A / 4B | 6/18 | +++ / +++ |
| 5A / 5B | 6/18 | +++ / +++ |
| 6A / 6B | 4/19 | ++ / +++ |
| 7A / 7B | 3/19 | ++/++ |
| 8A / 8B | 3 / 20 | +/+ |
| 9A / 9B | 3 / 20 | (+) / + |
| 10A / 10B | 3 / 20 | (+) / (+) |
| 11A / 11B | 12 / 19 | ++ / ND |
| 12B | 12 | ND |
| 13B | 8 | ND |
| Avg. flux permeates 1-10 Example 1A / 1B | 6.6 / 18.5 | |

### Example 2: Ultrafiltration and diafiltration of potato fruit juice with subsequent isolation of polyphenols and glycoalkaloids

30 L potato fruit juice, produced as described in sections *'Materials'* and *'Assays'* without any pretreatment to clarify the potato fruit juice, was subjected to ultrafiltration followed by diafiltration. The potato fruit juice, having a true protein concentration of 11.2 g/l, had a pH of 6.0 and a conductivity of 13.3 mS/cm. When the potato fruit juice was diluted with water to a 5 mg/ml true protein concentration and centrifuged in a tabletop centrifuge at 4000 G for 30 minutes, there remained a pellet constituting approximately 1 vol/vol% of the potato fruit juice volume. Accordingly, the potato fruit juice comprised a considerable amount of insoluble fibers. O.D. 600 nm of the potato fruit juice was 1.95 (measured as diluted 3X in 50 mM potassium phosphate, at pH 7.0. The resulting absorbance reading was multiplied by 3).

The ultrafiltration and diafiltration permeates obtained were mixed and thereafter subjected to nanofiltration for separation of the polyphenols and glycoalkaloids from salts. Hereafter the polyphenols were adsorbed onto an ion exchanger to separate polyphenols and glycoalkaloids.

### Ultrafiltration

The procedure was as follows: a 3 inch ROMICON PM30, 1.1 mm i.d. (Koch Membrane Systems, USA) hollow fiber membrane unit was employed. This unit has a membrane area of 2.3 m². The cross flow was set at 34 L/min and the TMP (transmembrane pressure) was regulated to be approximately 1.2 bar.

The 30 L potato fruit juice was concentrated using ultrafiltration to a retentate volume of 6 L (*i.e.* the concentration factor was 5X) and the 24 L clear purple/brown permeate (labelled *'first permeate'*) was collected and stored at 2-4 °C until further processing. Using the built-in heat exchanger of the equipment, the temperature of the retentate (labelled *'first retentate'*) was kept in the range of 20-25 °C during the entire ultrafiltration procedure.

The flux observed in the beginning of the ultrafiltration process was approximately 35 LMH and thereafter gradually decreased to 15 LMH when the retentate was 5X concentrated. There were no signs of membrane fiber clogging due the high content of insoluble material (insoluble fibers).

### Diafiltration

Following the 5X concentration using ultrafiltration, 6 L, of 3 g/L sodium sulfite was added to the first retentate (Sigma Aldrich USA, cat. No.: 13471), conductivity = 4.1 mS/cm at 18 °C, followed by further ultrafiltration to again reduce the retentate volume to 6 L while the corresponding permeate was collected in a separate vessel labelled *'diafiltrate'.* This procedure was repeated in total 12 times such that in total 72 L diafiltrate was obtained. However, only the first 36 L permeate were collected and stored for the subsequent nanofiltration step. Following the diafiltration with sodium sulfite solution, an additional 3 x 6 L water was added to remove the sodium sulfite from the retentate. The retentate (5.8 L) after diafiltration was labelled *'retentate'.*

During diafiltration, a gradual increase of flux was observed and at the final stages the flux was in the range of 25 LMH when a 6 L diafiltration portion was added. This correlated well with a gradual increase of pH in the retentate for each diafiltration step added which initially had a pH of 5.9 and at the end of diafiltration with sodium sulfite had a pH of 8.3.

Analysis of the first 6 x 6 L *'diafiltrate'* for alkaline colored phenolic compounds showed that the concentration of phenolic compounds was very high in the first permeate fractions and then gradually decreased for each 6 L permeate fraction. The sixth permeate fraction was estimated to contain less than 10 % phenolic compounds than the first permeate. Also, the majority of the total glycoalkaloids were analyzed to be in the first six permeate fractions, although still a significant concentration was also found in the subsequent fractions.

A sample of the *'retentate'* obtained after diafiltration was diluted 20 times in 50 mM potassium phosphate at pH 7.0 and the O.D. 600 of the diluted sample was measured to be 0.47. Thus, the undiluted retentate had an O.D. 600 nm of 20 x 0.47 = 9.4. The undiluted retentate had a pH value of 7.8 and a conductivity of 0.8 mS/cm.

The dry matter concentration of the retentate was measured to be 6.7 %, corresponding to a total protein/insoluble fiber product yield of 5.8 x 67 = 388.6 g. The true protein content was determined by the Kjeldahl method to be 87 % and the total glycoalkaloid content was determined to be 75 mg/kg protein (on a dry matter basis).

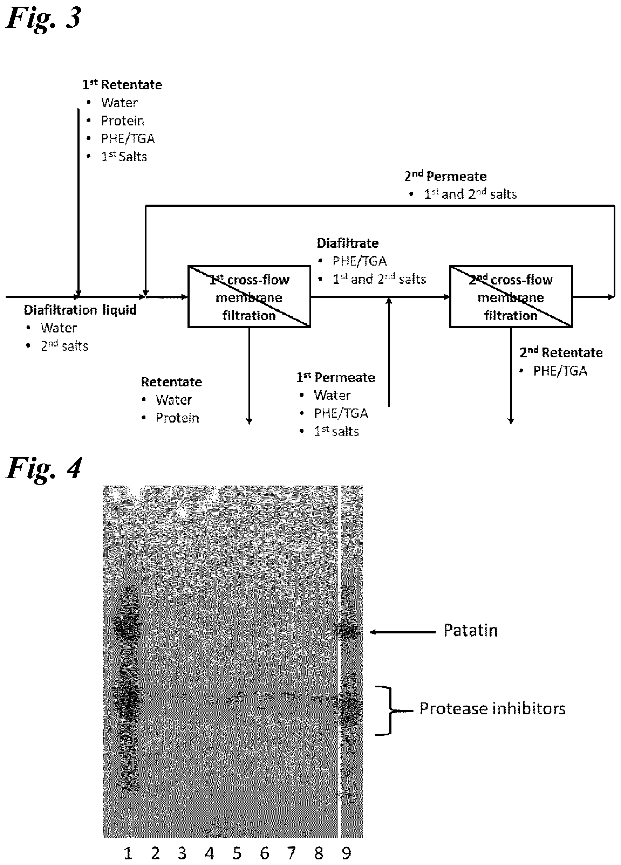

SDS-PAGE analysis of potato fruit juice, diafiltration permeates and the retentate after diafiltration was carried out as described in the *'Assays'* section. Results are depicted in Figure 4 (lane 1: potato fruit juice; lanes 2-8: diafiltrates step 1-7; lane 9: retentate after diafiltration). The analysis illustrates that the final protein product in the retentate after diafiltration (lane 9) has substantially the same protein composition as the starting material, *i.e.* potato fruit juice (lane 1). The analysis further illustrates that only very small amounts of potato protein migrates into the diafiltration permeates.

### Nanofiltration and separation of polyphenols and glycoalkaloids by ion exchange

A) The membrane filtration unit was equipped with a spiral wound nanofiltration membrane having an approximate molecular weight cut-off of 600-800 Dalton (Model NFG 2B 2538, Synder Filtration, USA). The *'first permeate'* and the *'diafiltrate'* obtained as described above were mixed to create a total volume of 60 L of a clear purple/brown solution containing the polyphenols and glycoalkaloids isolated from the potato fruit juice during ultrafiltration and diafiltration. The resulting solution had a pH of 6.8 and a conductivity of 10.4 mS/cm. 10 L of the mixed solution was then nanofiltered using a cross-flow of 22 L/min and a TMP of 5 bar. The process temperature as maintained at 20-25 °C. Samples of the permeate and the corresponding retentate were collected when 2 L permeate had been produced, were analyzed for conductivity and were tested for alkaline colored phenolic compounds as described in the *'Assays'* section. The nanofiltration process was conducted until the 10 L mixed solution was concentrated to about 3 L and then stopped.
   The conductivity of the collected nanofiltration permeate and retentate samples were found to be approximately 6.6 mS/cm and 10.9 mS/cm, respectively. This indicates that most of the salts present in the mixture passed the nanofiltration membrane into the permeate. The semi-quantitative test for alkaline colored phenolic compounds indicated that approx. 65 % of the alkaline colored phenolic compounds were retained in the retentate.
(B) The membrane filtration unit was then equipped with a spiral wound nanofiltration membrane having an approximate molecular weight cut-off of 300-500 Dalton (Model NFW 2B 1812F, Synder Filtration, USA), and another portion of 10 L of the mixed solution was then nanofiltered using a cross-flow of 6 L/min and a TMP of 5 bar. The process temperature was maintained at 20-25 °C. Samples of the permeate and the corresponding retentate were collected when 2 L permeate had been produced and were analyzed for conductivity, alkaline colored phenolic compounds and total glycoalkaloids as described in the *'Assays'* section. The nanofiltration process was conducted until the 10 L mixed solution was concentrated to about 2 L and then 2 L water was added with subsequent reconcentration to 2 L retentate. This was repeated once to obtain in total 12 L nanofiltration permeate.

The conductivity of the collected nanofiltration permeate and retentate samples were found to be approximately 4 mS/cm and 11.2 mS/cm, respectively. This indicates that approximately one third of the salts present in the mixture passed the membrane into the permeate. A significant additional part of the remaining salts were found to pass the membrane during the 2 diafiltration steps with water, which indicates that the polyphenols and total glycoalkaloids can be efficiently separated from the salts in the mixed solution. The semi-quantitative test for alkaline colored phenolic compounds as well as the test for total glycoalkaloids indicated that no detectable amounts of the alkaline colored phenolic compounds nor total glycoalkaloids were passing the membrane which indicated that this nanofiltration membrane retains approximately 100 % of the alkaline colored phenolic compounds and the total glycoalkaloids in the retentate.

The quality of the obtained permeate enables recycling of the permeate and thereby reuse of the water and salts as diafiltration liquid in the diafiltration step to create the *'diafiltrate'* after 5X concentration of the potato fruit juice as described above. In this way the total water and salt usage of the process according to the invention will be highly reduced.

### Separation of phenolic compounds and total glycoalkaloids by ion exchange

The partly desalted nanofiltration retentate from experiment (B) as described above was applied to a chromatographic column packed with Q Sepharose Fast Flow (Cat no. Q1126, Sigma Aldrich, USA) which was pre-washed and equilibrated with water. The pH of the nanofiltration retentate sample was 6.8. The sample was loaded until the column was nearly completely saturated with the bound phenolic compounds. The run-through (non-bound fraction) from the column was collected and analyzed for the content of alkaline colored phenolic compounds.

The phenolic compounds were found to bind strongly to the Q Sepharose Fast Flow adsorbent, which could be observed by visual inspection of the column in the form of a dark-brown band of bound phenols that appeared at the top and gradually filled the column as more sample was applied. The collected run-through was negative in the test for alkaline colored phenolic compounds, which indicates that substantially all phenolic compounds were bound the adsorbent. While the phenolic compounds can be negatively charged at near-neutral to alkaline pH values, the glycoalkaloids will be neutral or slightly positively charged and do therefore not bind to the adsorbent. Accordingly, they will migrate in the run-through fraction, and are thus separated from the phenolic compounds. The bound phenolic compounds could be efficiently released from the adsorbent with 0.1 M sodium hydroxide.

### Comparative Example 3: Ultrafiltration and diafiltration of potato fruit juice with an integrated step for inactivation of enzymatic activity

30 L potato fruit juice, produced as described in sections *'Materials'* and *'Assays'* without any pretreatment, was subjected to ultrafiltration and diafiltration to clarify the potato fruit juice. The potato fruit juice, having a true protein concentration of 11.5 g/l, had a pH of 6.0 and a conductivity of 13.1mS/cm. When the potato fruit juice was diluted with water to a 5 mg/ml true protein concentration and centrifuged in a tabletop centrifuge at 4000 G for 30 min there remained a pellet constituting approximately 1 vol/vol% of the potato fruit juice volume. Accordingly, the potato fruit juice comprised a considerable amount of insoluble fibers. O.D. 600 nm of the potato fruit juice was 2.0 (measured as diluted 3X in 50 mM potassium phosphate, at pH 7.0. The resulting absorbance reading was multiplied by 3).

The retentate obtained after a first diafiltration step was subjected to an enzyme inactivation step, followed by further diafiltration, solubilization and final purification of the resulting protein product.

### Ultrafiltration

The procedure was as follows: a 3 inch ROMICON PM30, 1.1 mm i.d. (Koch Membrane Systems, USA) hollow fiber membrane unit was employed. This unit has a membrane area of 2.3 m². The cross flow was set at 34 L/min and the TMP (transmembrane pressure) was regulated to be approximately 1.2 bar.

The 30 L potato fruit juice was concentrated using ultrafiltration to a retentate volume of 6 L (*i.e.* the concentration factor was 5X) and the 24 L clear purple/brown permeate (labelled *'first permeate'*) was collected and stored at 2-4 °C until further processing. Using the built-in heat exchanger of the equipment the temperature of the retentate (labelled *'first retentate'*) was kept in the range of 20-25 °C during the entire ultrafiltration procedure.

The flux observed in the beginning of the ultrafiltration process was approximately 35 LMH and thereafter gradually decreased to 15 LMH when the retentate was 5X concentrated. There were no signs of membrane fiber clogging due the high content of insoluble material (insoluble fibers).

### Diafiltration

Following the 5X concentration using ultrafiltration, 6 L, 3 g/L sodium sulfite was added to the first retentate (Sigma Aldrich USA, cat. No.: 13471), conductivity = 4.1 mS/cm at 18 °C, followed by further ultrafiltration to again reduce the retentate volume to 6 L while the corresponding permeate was collected in a separate vessel labelled *'diafiltrate'.* This procedure was repeated in total 6 times such that in total 36 L diafiltrate was obtained. The retentate was labelled *'retentate'.*

A sample of the *'retentate'* obtained after diafiltration was diluted 20 times in 50 mM potassium phosphate at pH 7.0 and the O.D. 600 of the diluted sample was measured to be 0.6. Thus, the undiluted retentate had an O.D. 600 nm of 20 x 0.6 = 12. The undiluted retentate had a pH value of 7.7.

During diafiltration a gradual increase of flux was observed and at the final stages the flux was in the range of 22 LMH when a 6 L diafiltration portion was added. This correlated well with a gradual increase of pH in the retentate for each diafiltration step added which initially had a pH of 5.9 and at the end of this first diafiltration with sodium sulfite had a pH of 7.7.

Analysis of the first 6 x 6 L diafiltration permeate for alkaline colored phenols showed that the concentration of phenols was very high in the first permeate fractions and then gradually decreased for each 6 L permeate fraction. The sixth permeate fraction was estimated to contain less than 10 % of the initial permeates. Also, the majority of the glycoalkaloids were analyzed to be in the first six permeate fractions, although still a significant concentration was also found in the subsequent fractions.

### Inactivation of enzyme activity, further diafiltration, pH adjustment and final diafiltration

A 20 ml sample of the retentate obtained after the first diafiltration step was divided into two aliquots which were adjusted with sulfuric acid to a pH of 4.0 and 3.0, respectively. The two solutions were then again divided into two aliquots which were incubated at room temperature (RT, 20-23 °C) and 40 °C, respectively, for 3 hrs. During the incubation, samples for analysis of esterase activity were withdrawn, neutralized with sodium hydroxide and analyzed for esterase activity as described in the *'Assays'* section.

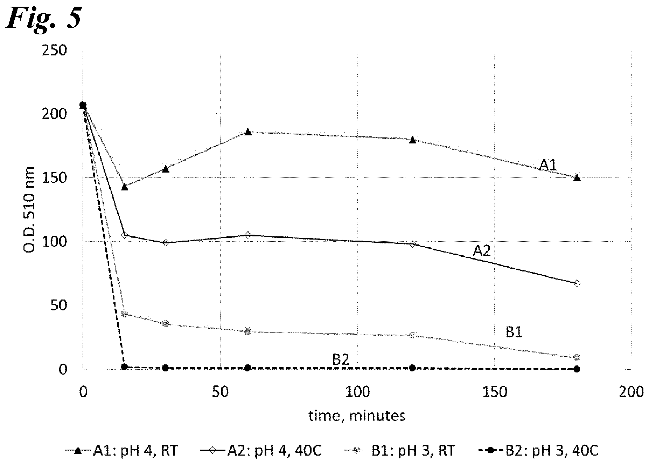

Figure 5 shows the esterase activity signal (O.D.510 nm) in the different samples as a function of incubation time at pH 4.0 and pH 3.0, respectively.

Figure 5 illustrates that the esterase activity in the retentate sample is reduced when incubated at pH 4.0 or 3.0, respectively, at room temperature. At pH 3, the esterase activity signal at 510 nm decreases strongly over time and after 3 hrs., it is reduced with a factor of 24. When the temperature is increased to 40 °C, the inactivation is very effective even after 15 minutes, after which the esterase activity is reduced with a factor of 98. At room temperature and pH 3.0, it is observed that an activity drop of about 85 % is reached after about 1 hour and that this level is rather constant until up to about 2 hours after incubation. From a process robustness perspective, it may be preferable to have ample time to perform the operations without changes of the process outcome and it was therefore chosen to perform the next preparative steps at pH 3.0 and room temperature.

### Preparative inactivation procedure and subsequent solubilization

The retentate after diafiltration was kept in the membrane filtration unit and 6 L, 20 mM sodium sulfate was added (SL 99.8 % from Lenzing, Austria adjusted to pH 3.3 with sulfuric acid, conductivity = 3.8 mS/cm at 20 °C). Thereafter, the pH was adjusted to 3.0 with sulfuric acid while recirculating in the membrane filtration unit using the built-in heat exchanger to keep the temperature in the range of 20-25 °C. Following recirculation for 15 minutes, the retentate was then again concentrated to a volume of 6 L retentate. The permeate was collected and labelled 'acidic permeate' and stored at 2-4 °C. Following the concentration, again 6 L, 20 mM sodium sulfate, pH 3.3, was added to the retentate. This procedure was repeated in total 6 times such that in total 42 L acidic permeate was obtained.

To the retentate was then added 6 L water and the pH was adjusted to pH 8.5 with sodium hydroxide while the retentate was kept in the membrane filtration under recirculation. A total of 2 hours at 20-25 °C was passing from the time the retentate was adjusted to pH 3.0 and until the pH was raised again to pH 8.5. The retentate was then again concentrated to a volume of 6 L retentate. The permeate was collected and labelled *'finalpermeate'* and stored at 2-4 °C. Following the concentration, 6 L of water was added to the retentate. This procedure was repeated in total 3 times such that in total 24 L final permeate was obtained. The retentate was collected from the membrane filtration unit in a volume of 5.8 L and labelled *'finalretentate'.*

The conductivity of the final retentate was 0.8 mS (at 20 °C). The pH was 7.9. The dry matter concentration of the final retentate was 6.5 %, corresponding to a total yield of dry matter of 377 g. The protein purity was determined by Kjeldahl analysis as described in the methods section to be 89 % and the total glycoalkaloid content was determined to be 25 mg/kg protein (on a dry matter basis). Testing for alkaline colored phenolic compounds gave no visible reaction, indicating that practically all phenolic compounds were removed.

When the final retentate was diluted to a true protein concentration equal to the potato fruit juice (11.5 g/L) and was analyzed for esterase activity as described in the *'Assays'* section, it was found that the activity signal was reduced with approximately 85 %. Likewise, when performing the determination of polyphenol oxidase (PPO) activity no visible reaction was observed, indicating that practically all the PPO activity was eliminated.

A sample of the final retentate was diluted 20 times in 50 mM potassium phosphate at pH 7.0 and the O.D. 600 of the diluted sample was measured to be 0.55. Thus, the undiluted final retentate had an O.D. 600 nm of 20 x 0.55 = 11. Accordingly, the final retentate comprised a considerable amount of insoluble fibers.

When diluted to a dry matter concentration of 0.5 % in water and after centrifugation for 30 minutes at 4000 G, there was less than 1 vol/vol% pellet. The dry matter of the supernatant was still close to 0.5 % indicating a more than 90 % solubility of the potato protein product. This is in sharp contrast to prior art reports indicating that low pH treatment of potato fruit juice leads to significant loss of solubility, and thereby functionality.

## Claims

1. A method for the separation of potato proteins from one or more first salts and phenolic and/or glycoalkaloid compounds in potato fruit juice or a derivative thereof, said method comprising the steps of:
(i) providing a potato fruit juice or a derivative thereof comprising potato proteins, one or more first salts and phenolic and/or glycoalkaloid compounds;
(ii) subjecting said potato fruit juice or the derivative thereof to a first cross-flow membrane filtration process wherein at least a portion of the first salts and at least a portion of the phenolic and/or glycoalkaloid compounds migrate across the membrane into a first permeate and the potato proteins are retained in a first retentate;
(iii) subjecting the first retentate of step (ii) to a diafiltration step comprising adding one or more second salts and water to the first retentate, while continuing the membrane filtration process, to create a diafiltrate containing at least a portion of said phenolic and/or glycoalkaloid compounds and the added second salts and a retentate; and
(iv) subjecting the first permeate and/or said diafiltrate from said first cross-flow membrane filtration process to a second cross-flow membrane filtration process wherein at least a portion of the salts present therein migrate across the membrane into a second permeate and the phenolic and/or glycoalkaloid compounds are retained in a second retentate.

2. The method according to claim 1, further comprising the step (v) of:
subjecting the first retentate obtained in step (iii) to a microfiltration membrane filtration process wherein at least a portion of the potato proteins soluble in said first retentate migrate across the membrane into a microfiltration permeate with a lower turbidity than the turbidity of said first retentate.

3. The method according to claim 1 or 2, wherein the second permeate is used, at least in part, as the source of said one or more second salts and water in step (iii).

4. The method according to any one of the preceding claims, further comprising the step of adjusting the pH of said potato fruit juice or the derivative thereof to a pH in the range 4.5 to 8.5, such as in the range of 5.2 to 7.5, preferably in the range of 4.5 to 6.5, such as in the range of 4.8 to 6.5, or 5.0 to 6.5 prior to said first cross-flow membrane filtration process and/or; further comprising the step of adjusting the conductivity of said potato fruit juice or the derivative thereof to a conductivity in the range of 1 to 50 mS/cm, 2 to 40 mS/cm, 3 to 30 mS/cm, 3 to 25 mS/cm, 3 to 20 mS/cm, or 5 to 17 mS/cm.

5. The method according to any one of the preceding claims, wherein the potato proteins are selected from the group consisting of patatin, protease inhibitors, lipoxygenase, polyphenol oxidase, acid and alkaline phosphatase, or mixtures thereof, preferably patatin or protease inhibitors, or mixtures thereof.

6. The method according to any one of the preceding claims, wherein the potato fruit juice or the derivative thereof is pretreated by centrifugation and/or filtration to remove insoluble material of a particle size larger than 10 micron prior to said first cross-flow membrane filtration process.

7. The method according to any one of the preceding claims, further comprising the step of continuing the addition of said one or more second salts and water to the first retentate in step (iii) while continuing the membrane filtration process until the first retentate contains less than a target amount of the phenolic and/or total glycoalkaloid compounds, whereby the separation of the potato proteins from phenolic and/or total glycoalkaloid compounds has been achieved, wherein the target amount of remaining phenolic and/or total glycoalkaloid compounds in the first retentate corresponds to less than 5000 mg phenolic and/or total glycoalkaloid compounds per kg potato protein on the basis of dry weight, such as less than 4000 mg/kg, less than 3000 mg/kg, less than 2000 mg/kg, less than 1500 mg/kg, less than 1250 mg/kg, less than 1000 mg/kg, less than 750 mg/kg, less than 500 mg/kg, or less than 200 mg phenolic and/or total glycoalkaloid compounds/kg potato protein on the basis of dry weight.

8. The method according to claim 7, wherein the amount of phenolic and/or total glycoalkaloid compounds having a molecular weight below 10 kDa remaining in the first retentate in step (iii) is less than 1500 mg/kg, such as less than 1000 mg/kg, less than 750 mg/kg, less than 500 mg/kg, less than 300 mg/kg, less than 200 mg/kg, less than 100 mg/kg, as less than 50 mg/kg, measured as mg phenolic and/or total glycoalkaloid compounds/kg potato protein on the basis of dry weight.

9. The method according to any one of claims 2-8, wherein the microfiltration permeate obtained in step (v) is further treated with a third cross-flow membrane filtration process whereby the potato protein is concentrated in a third retentate and the salts migrate through the membrane to create a third permeate and optionally wherein the third permeate is used as a diafiltration liquid to be added during the microfiltration process in step (v) to wash out further potato proteins from the first retentate into the microfiltration permeate.

10. The method according to any one of the previous claims, wherein the one or more second salts are selected from sodium, potassium, ammonium, calcium or magnesium salts of a mineral acid or an organic acid and/or;
wherein the one or more second salts are selected from salts of monovalent inorganic anions and cations, such as sodium chloride, potassium chloride, ammonium chloride;
preferably wherein the one or more salts are selected from sodium sulfate and potassium sulfate.

11. The method according to any one of the preceding claims,
wherein the conductivity of the first retentate remains within the range of 1 to 50 mS/cm, such as in the range of 2 to 40 mS/cm, in the range of 3 to 30 mS/cm, in the range of 3 to 25 mS/cm, in the range of 3 to 20 mS/cm, in the range of 5 to 17 mS/cm, in the range of 6 to 25 mS/cm, in the range of 7 to 25 mS/cm, or in the range of 10 to 25 mS/cm during step step (iii), preferably during step (ii) and step (iii) and/or;
wherein the pH of the first retentate remains within the range of 4.5 to 8.5, such as in the range of 5.2 to 7.5, preferably in the range of 4.5 to 6.5, such as in the range of 4.8 to 6.5, or in the range of 5.0 to 6.5 during step (ii) and step (iii) and/or;
wherein the first retentate remains at a temperature in the range of 1 to 60 °C, such as 5 to 55 °C, 10 to 50 °C, 15 to 48 °C, 12 to 45 °C, 18 to 30 °C, or 22 to 28 °C during step (ii) and step (iii)

12. The method according to any one of the preceding claims, wherein the addition of the one or more second salts and water in step (iii) is performed continuously and at the same flow rate as the diafiltrate migrates through the membrane and/or;
wherein the addition of the one or more second salts and water in step (iii) is performed in portions, such as portions approximately equal in volume to the volume of the first retentate.

13. The method according to any one of the preceding claims, wherein the total volume of water added during diafiltration step (iii) is in the range of 4 to 20 times the volume of the first retentate, such as 4 to 15 times, 5 to 12 times, 5 to 10 times, 6 to 10 times, 7 to 9 times, or 5 to 9 times the volume of the first retentate.

14. The method according to any one of the preceding claims, wherein step (iii) comprises a second phase of diafiltration following the addition of one or more second salts and water wherein the diafiltration is continued with the addition of water without adding any further salts, preferably wherein the second phase of diafiltration is continued with water and without the addition of further salts until the conductivity of the retentate is less than 10 mS/cm, such as less than 8 mS/cm, less than 5 mS/cm, less than 4 mS/cm, less than 3 mS/cm, less than 2 mS/cm, or less than 1 mS/cm.

15. The method according to any one of the preceding claims, further comprising a germicidal step at a temperature in the range of 2-60 °C, such as in the range of 5-55 °C, 8-52 °C, 10-50 °C, 12-45 °C, 12-25 °C, or 12-18 °C, using high intensity UV light, X-rays, pulsed electric field treatment or high-pressure pasteurization.

## Patentansprüche

1. Verfahren zur Abtrennung von Kartoffelproteinen von einem oder mehreren ersten Salzen und phenolischen und/oder glycoalkaloidischen Verbindungen in Kartoffelfruchtsaft oder einem Derivat davon, wobei das Verfahren die folgenden Schritte umfasst:
(i) Bereitstellen eines Kartoffelfruchtsafts oder eines Derivats davon, das Kartoffelproteine, ein oder mehrere erste Salze und phenolische und/oder glycoalkaloidische Verbindungen umfasst;
(ii) Unterziehen des Kartoffelfruchtsafts oder des Derivats davon einem ersten Querstrom-Membranfiltrationsprozess, bei dem mindestens ein Teil der ersten Salze und mindestens ein Teil der phenolischen und/oder glycoalkaloidischen Verbindungen durch die Membran in ein erstes Permeat wandern und die Kartoffelproteine in einem ersten Retentat zurückgehalten werden;
(iii) Unterziehen des ersten Retentats aus Schritt (ii) einem Diafiltrationsschritt, der das Hinzufügen eines oder mehrerer zweiter Salze und Wasser zu dem ersten Retentat umfasst, während der Membranfiltrationsprozess fortgesetzt wird, um ein Diafiltrat zu erzeugen, das mindestens einen Teil der phenolischen und/oder glycoalkaloidischen Verbindungen und die hinzugefügten zweiten Salze und ein Retentat enthält; und
(iv) Unterziehen des ersten Permeats und/oder des Diafiltrats aus dem ersten Querstrom-Membranfiltrationsprozess einem zweiten Querstrom-Membranfiltrationsprozess, wobei mindestens ein Teil der darin vorhandenen Salze durch die Membran in ein zweites Permeat wandert und die phenolischen und/oder glycoalkaloidischen Verbindungen in einem zweiten Retentat zurückgehalten werden.

2. Verfahren nach Anspruch 1, weiter umfassend den folgenden Schritt (v):
Unterziehen des in Schritt (iii) erhaltenen ersten Retentats einem Mikrofiltrations-Membranfiltrationsprozess, bei dem mindestens ein Teil der in dem ersten Retentat löslichen Kartoffelproteine durch die Membran wandert in ein Mikrofiltrationspermeat mit einer geringeren Trübung als der Trübung des ersten Retentats.

3. Verfahren nach Anspruch 1 oder 2, wobei das zweite Permeat zumindest teilweise als Quelle für das eine oder die mehreren zweiten Salze und Wasser in Schritt (iii) verwendet wird.

4. Verfahren nach einem der voranstehenden Ansprüche, weiter umfassend den Schritt des Einstellens des pH-Werts des Kartoffelfruchtsafts oder dessen Derivats auf einen pH-Wert im Bereich von 4,5 bis 8,5, beispielsweise im Bereich von 5,2 bis 7,5, vorzugsweise im Bereich von 4,5 bis 6,5, beispielsweise im Bereich von 4,8 bis 6,5 oder 5,0 bis 6,5, vor dem ersten Querstrom-Membranfiltrationsprozess, und/oder weiter umfassend den Schritt des Einstellens der Leitfähigkeit des Kartoffelfruchtsafts oder dessen Derivats auf eine Leitfähigkeit im Bereich von 1 bis 50 mS/cm, 2 bis 40 mS/cm, 3 bis 30 mS/cm, 3 bis 25 mS/cm, 3 bis 20 mS/cm oder 5 bis 17 mS/cm.

5. Verfahren nach einem der voranstehenden Ansprüche, wobei die Kartoffelproteine aus der Gruppe ausgewählt sind, die aus Patatin, Proteaseinhibitoren, Lipoxygenase, Polyphenoloxidase, saurer und alkalischer Phosphatase oder Mischungen davon besteht, vorzugsweise Patatin oder Proteaseinhibitoren oder Mischungen davon.

6. Verfahren nach einem der voranstehenden Ansprüche, wobei der Kartoffelfruchtsaft oder dessen Derivat durch Zentrifugation und/oder Filtration vorbehandelt wird, um unlösliches Material mit einer Partikelgröße von mehr als 10 Mikrometern zu entfernen vor dem ersten Querstrom-Membranfiltrationsprozess.

7. Verfahren nach einem der voranstehenden Ansprüche, weiter umfassend den Schritt des Fortsetzens der Zugabe des einen oder der mehreren zweiten Salze und Wasser zu dem ersten Retentat in Schritt (iii), während der Membranfiltrationsprozess fortgesetzt wird, bis das erste Retentat weniger als eine Zielmenge der phenolischen und/oder der gesamten glycoalkaloidischen Verbindungen enthält, wodurch die Trennung der Kartoffelproteine von den phenolischen und/oder den gesamten glycoalkaloidischen Verbindungen erreicht wird, wobei die Zielmenge der verbleibenden phenolischen und/oder der gesamten glycoalkaloidischen Verbindungen in dem ersten Retentat weniger als 5.000 mg phenolischen und/oder der gesamten glycoalkaloidischen Verbindungen pro kg Kartoffelprotein auf Trockengewichtsbasis, wie beispielsweise weniger als 4.000 mg/kg, weniger als 3.000 mg/kg, weniger als 2.000 mg/kg, weniger als 1.500 mg/kg, weniger als 1.250 mg/kg, weniger als 1.000 mg/kg, weniger als 750 mg/kg, weniger als 500 mg/kg oder weniger als 200 mg phenolischen und/oder der gesamten glycoalkaloidischen Verbindungen/kg Kartoffelprotein auf Trockengewichtsbasis entspricht.

8. Verfahren nach Anspruch 7, wobei die Menge an phenolischen und/oder der gesamten glycoalkaloidischen Verbindungen mit einem Molekulargewicht unter 10 kDa, die in dem ersten Retentat in Schritt (iii) verbleiben, weniger als 1.500 mg/kg beträgt, beispielsweise weniger als 1.000 mg/kg, weniger als 750 mg/kg, weniger als 500 mg/kg, weniger als 300 mg/kg, weniger als 200 mg/kg, weniger als 100 mg/kg, weniger als 50 mg/kg, gemessen als mg phenolischer und/oder der gesamten glycoalkaloidischen Verbindungen/kg Kartoffelprotein auf Trockengewichtsbasis.

9. Verfahren nach einem der Ansprüche 2-8, wobei das in Schritt (v) erhaltene Mikrofiltrationspermeat weiter mit einem dritten Querstrom-Membranfiltrationsprozess behandelt wird, wodurch das Kartoffelprotein in einem dritten Retentat konzentriert wird und die Salze durch die Membran wandern, um ein drittes Permeat zu erzeugen, und wobei das dritte Permeat optional als Diafiltrationsflüssigkeit verwendet wird, die während des Mikrofiltrationsprozesses in Schritt (v) hinzugefügt wird, um weitere Kartoffelproteine aus dem ersten Retentat in das Mikrofiltrationspermeat auszuwaschen.

10. Verfahren nach einem der voranstehenden Ansprüche, wobei das eine oder die mehreren zweiten Salze aus Natrium-, Kalium-, Ammonium-, Calcium- oder Magnesiumsalzen einer mineralischen Säure oder einer organischen Säure ausgewählt sind und/oder;
wobei das eine oder die mehreren zweiten Salze aus Salzen monovalenter anorganischer Anionen und Kationen ausgewählt sind, wie Natriumchlorid, Kaliumchlorid, Ammoniumchlorid;
wobei das eine oder die mehreren Salze vorzugsweise aus Natriumsulfat und Kaliumsulfat ausgewählt sind.

11. Verfahren nach einem der voranstehenden Ansprüche,
wobei die Leitfähigkeit des ersten Retentats während Schritt (iii), vorzugsweise während Schritt (ii) und Schritt (iii), innerhalb des Bereichs von 1 bis 50 mS/cm bleibt, beispielsweise im Bereich von 2 bis 40 mS/cm, im Bereich von 3 bis 30 mS/cm, im Bereich von 3 bis 25 mS/cm, im Bereich von 3 bis 20 mS/cm, im Bereich von 5 bis 17 mS/cm, im Bereich von 6 bis 25 mS/cm, im Bereich von 7 bis 25 mS/cm oder im Bereich von 10 bis 25 mS/cm, und/oder;
wobei der pH-Wert des ersten Retentats während Schritt (ii) und Schritt (iii) im Bereich von 4,5 bis 8,5 bleibt, beispielsweise im Bereich von 5,2 bis 7,5, vorzugsweise im Bereich von 4,5 bis 6,5, beispielsweise im Bereich von 4,8 bis 6,5, oder im Bereich von 5,0 bis 6,5, und/oder;
wobei das erste Retentat während Schritt (ii) und Schritt (iii) bei einer Temperatur im Bereich von 1 bis 60 °C, beispielsweise 5 bis 55 °C, 10 bis 50 °C, 15 bis 48 °C, 12 bis 45 °C, 18 bis 30 °C oder 22 bis 28 °C verbleibt.

12. Verfahren nach einem der voranstehenden Ansprüche, wobei die Zugabe des einen oder der mehreren zweiten Salze und des Wassers in Schritt (iii) kontinuierlich und mit derselben Durchflussrate durchgeführt wird, mit der das Diafiltrat durch die Membran wandert, und/oder
wobei die Zugabe des einen oder der mehreren zweiten Salze und des Wassers in Schritt (iii) in Portionen durchgeführt wird, beispielsweise in Portionen, deren Volumen ungefähr dem Volumen des ersten Retentats entspricht.

13. Verfahren nach einem der voranstehenden Ansprüche, wobei das Gesamtvolumen des während des Diafiltrationsschritts (iii) zugegebenen Wassers im Bereich des 4- bis 20-fachen Volumens des ersten Retentats liegt, beispielsweise des 4- bis 15-fachen, 5-bis 12-fachen, 5- bis 10-fachen, 6- bis 10-fachen 7- bis 9-fachen oder 5- bis 9-fachen des Volumens des ersten Retentats.

14. Verfahren nach einem der voranstehenden Ansprüche, wobei Schritt (iii) eine zweite Phase der Diafiltration nach der Zugabe eines oder mehrerer zweiter Salze und von Wasser umfasst, wobei die Diafiltration mit der Zugabe von Wasser ohne Zugabe weiterer Salze fortgesetzt wird, wobei die zweite Phase der Diafiltration vorzugsweise mit Wasser und ohne Zugabe weiterer Salze fortgesetzt wird, bis die Leitfähigkeit des Retentats weniger als 10 mS/cm beträgt, beispielsweise weniger als 8 mS/cm, weniger als 5 mS/cm, weniger als 4 mS/cm, weniger als 3 mS/cm, weniger als 2 mS/cm oder weniger als 1 mS/cm.

15. Verfahren nach einem der voranstehenden Ansprüche, das weiter einen keimtötenden Schritt bei einer Temperatur im Bereich von 2-60 °C, beispielsweise im Bereich von 5-55 °C, 8-52 °C, 10-50 °C, 12-45 °C, 12-25 °C oder 12-18 °C umfasst, unter Verwendung von hochintensivem UV-Licht, Röntgenstrahlen, gepulster elektrischer Feldbehandlung oder Hochdruckpasteurisierung.

## Revendications

1. Procédé pour la séparation de protéines de pomme de terre d'un ou plusieurs premiers sels et de composés phénoliques et/ou glycoalcaloïdes dans du jus de pomme de terre ou un dérivé de celui-ci, ledit procédé comprenant les étapes suivantes :
(i) fournir un jus de pomme de terre ou un dérivé de celui-ci comprenant des protéines de pomme de terre, un ou plusieurs premiers sels et des composés phénoliques et/ou glycoalcaloïdes ;
(ii) soumettre ledit jus de pomme de terre ou le dérivé de celui-ci à un premier processus de filtration sur membrane à flux tangentiel où au moins une partie des premiers sels et au moins une partie des composés phénoliques et/ou glycoalcaloïdes migrent à travers la membrane dans un premier perméat et les protéines de pomme de terre sont retenues dans un premier rétentat ;
(iii) soumettre le premier rétentat de l'étape (ii) à une étape de diafiltration comprenant l'ajout d'un ou plusieurs deuxièmes sels et d'eau au premier rétentat, tout en poursuivant le procéssus de filtration sur membrane, afin de créer un diafiltrat contenant au moins une partie desdits composés phénoliques et/ou glycoalcaloïdes et les deuxièmes sels ajoutés, et un rétentat ; et
(iv) soumettre le premier perméat et/ou ledit diafiltrat provenant dudit premier processus de filtration sur membrane à flux tangentiel à un deuxième processus de filtration sur membrane à flux tangentiel où au moins une partie des sels présents dans celui-ci migrent à travers la membrane dans un deuxième perméat et les composés phénoliques et/ou glycoalcaloïdes sont retenus dans un deuxième rétentat.

2. Procédé selon la revendication 1, comprenant en outre l'étape (v) de:
soumettre le premier rétentat obtenu à l'étape (iii) à un processus de filtration sur membrane de microfiltration où au moins une partie des protéines de pomme de terre solubles dans ledit premier rétentat migrent à travers la membrane dans un perméat de microfiltration présentant une turbidité inférieure à la turbidité dudit premier rétentat.

3. Procédé selon la revendication 1 ou 2, où le deuxième perméat est utilisé, au moins en partie, comme la source desdits un ou plusieurs deuxièmes sels et d'eau à l'étape (iii).

4. Procédé selon l'une quelconque des revendications précédentes, comprenant en outre l'étape d'adjustement du pH dudit jus de pomme de terre ou du dérivé de celui-ci à un pH dans la plage de 4,5 à 8,5, tel que dans la plage de 5,2 à 7,5, de préférence dans la plage de 4,5 à 6,5, tel que dans la plage de 4,8 à 6,5, ou de 5,0 à 6,5 avant ledit premier processus de filtration sur membrane à flux tangentiel et/ou; comprenant en outre l'étape d'adjustement de la conductivité dudit jus de pomme de terre ou du dérivé de celui-ci à une conductivité dans la plage de 1 à 50 mS/cm, de 2 à 40 mS/cm, de 3 à 30 mS/cm, de 3 à 25 mS/cm, de 3 à 20 mS/cm, ou de 5 à 17 mS/cm.

5. Procédé selon l'une quelconque des revendications précédentes, où les protéines de pomme de terre sont choisies dans le groupe constitué par la patatine, des inhibiteurs de protéase, la lipoxygénase, la polyphénol oxydase, la phosphatase acide et alcaline, ou des mélanges de ceux-ci, de préférence la patatine ou des inhibiteurs de protéase, ou des mélanges de ceux-ci.

6. Procédé selon l'une quelconque des revendications précédentes, où le jus de pomme de terre ou le dérivé de celui-ci est prétraité par centrifugation et/ou filtration afin d'éliminer les matières insolubles dont la taille des particules est supérieure à 10 microns avant ledit premier processus de filtration sur membrane à flux tangentiel.

7. Procédé selon l'une quelconque des revendications précédentes, comprenant en outre l'étape consistant à poursuivre l'ajout desdits un ou plusieurs deuxièmes sels et d'eau au premier rétentat à l'étape (iii) tout en poursuivant le processus de filtration sur membrane jusqu'à ce que le premier rétentat contienne moins qu'une quantité cible de composés phénoliques et/ou glycoalcaloïdes totaux, ce qui permet d'obtenir la séparation des protéines de pomme de terre des composés phénoliques et/ou glycoalcaloïdes totaux, où la quantité cible de composés phénoliques et/ou glycoalcaloïdes totaux restants dans le premier rétentat correspond à moins de 5000 mg de composés phénoliques et/ou glycoalcaloïdes totaux par kg de protéines de pomme de terre sur la base du poids sec, telle que moins de 4000 mg/kg, moins de 3000 mg/kg, moins de 2000 mg/kg, moins de 1500 mg/kg, moins de 1250 mg/kg, moins de 1000 mg/kg, moins de 750 mg/kg, moins de 500 mg/kg ou moins de 200 mg de composés phénoliques et/ou glycoalcaloïdes totaux/kg de protéines de pomme de terre sur la base du poids sec.

8. Procédé selon la revendication 7, où la quantité de composés phénoliques et/ou glycoalcaloïdes totaux ayant un poids moléculaire inférieur à 10 kDa restant dans le premier rétentat à l'étape (iii) est inférieure à 1500 mg/kg, telle qu'inférieure à 1000 mg/kg, inférieure à 750 mg/kg, inférieure à 500 mg/kg, inférieure à 300 mg/kg, inférieure à 200 mg/kg, inférieure à 100 mg/kg, inférieure à 50 mg/kg, mesurée en mg de composés phénoliques et/ou glycoalcaloïdes totaux/kg de protéines de pomme de terre sur la base du poids sec.

9. Procédé selon l'une quelconque des revendications 2 à 8, où le perméat de microfiltration obtenu à l'étape (v) est traité en outre par un troisième processus de filtration sur membrane à flux tangentiel, grâce auquel la protéine de pomme de terre est concentrée dans un troisième rétentat et les sels migrent à travers la membrane pour créer un troisième perméat, et où, optionnellement, le troisième perméat est utilisé comme un liquide de diafiltration à ajouter pendant le processus de microfiltration à l'étape (v) afin d'extraire davantage de protéines de pomme de terre du premier rétentat dans le perméat de microfiltration.

10. Procédé selon l'une quelconque des revendications précédentes, où l'un ou plusieurs deuxièmes sels sont choisis parmi des sels de sodium, de potassium, d'ammonium, de calcium ou de magnésium d'un acide minéral ou d'un acide organique et/ou ;
où l'un ou plusieurs deuxièmes sels sont choisis parmi des sels d'anions et de cations inorganiques monovalents, tels que le chlorure de sodium, le chlorure de potassium, le chlorure d'ammonium ;
de préférence où l'un ou plusieurs sels sont choisis parmi le sulfate de sodium et le sulfate de potassium.

11. Procédé selon l'une quelconque des revendications précédentes,
où la conductivité du premier rétentat reste dans la plage de 1 à 50 mS/cm, telle que dans la plage de 2 à 40 mS/cm, dans la plage de 3 à 30 mS/cm, dans la plage de 3 à 25 mS/cm, dans la plage de 3 à 20 mS/cm, dans la plage de 5 à 17 mS/cm, dans la plage de 6 à 25 mS/cm, dans la plage de 7 à 25 mS/cm, ou dans la plage de 10 à 25 mS/cm pendant l'étape (iii), de préférence pendant l'étape (ii) et l'étape (iii) et/ou ;
où le PH du premier rétentat reste dans la plage de 4,5 à 8,5, tel que dans la plage de 5,2 à 7,5, de préférence dans la plage de 4,5 à 6,5, tel que dans la plage de 4,8 à 6,5, ou dans la plage de 5,0 à 6,5 pendant l'étape (ii) et l'étape (iii) et/ou ; où le premier rétentat reste à une température dans la plage de 1 à 60 °C, telle que 5 à 55 °C, 10 à 50 °C, 15 à 48 °C, 12 à 45 °C, 18 à 30 °C ou 22 à 28 °C pendant l'étape (ii) et l'étape (iii).

12. Procédé selon l'une quelconque des revendications précédentes, où l'ajout de l'un ou plusieurs deuxièmes sels et d'eau à l'étape (iii) est effectué en continu et au même débit que le diafiltrat migre à travers la membrane et/ou ;
où l'ajout de l'un ou plusieurs deuxièmes sels et d'eau à l'étape (iii) est effectué par portions, telles que des portions approximativement égales en volume au volume du premier rétentat,

13. Procédé selon l'une quelconque des revendications précédentes, où le volume total d'eau ajouté pendant l'étape de diafiltration (iii) est dans la plage de 4 à 20 fois le volume du premier rétentat, tel que 4 à 15 fois, 5 à 12 fois, 5 à 10 fois, 6 à 10 fois, 7 à 9 fois ou 5 à 9 fois le volume du premier rétentat.

14. Procédé selon l'une quelconque des revendications précédentes, où l'étape (iii) comprend une deuxième phase de diafiltration suivant l'ajout d'un ou plusieurs deuxièmes sels et d'eau, où la diafiltration est poursuivie avec l'ajout d'eau sans ajouter d'autres sels, de préférence où la deuxième phase de diafiltration est poursuivie avec de l'eau et sans ajout d'autres sels jusqu'à ce que la conductivité du rétentat soit inférieure à 10 mS/cm, telle que inférieure à 8 mS/cm, inférieure à 5 mS/cm, inférieure à 4 mS/cm, inférieure à 3 mS/cm, inférieure à 2 mS/cm ou inférieure à 1 mS/cm.

15. Procédé selon l'une quelconque des revendications précédentes, comprenant en outre une étape germicide à une température dans la plage de 2 à 60 °C, telle que dans la plage de 5 à 55 °C, 8 à 52 °C, 10 à 50 °C, 12 à 45 °C, 12 à 25 °C ou 12 à 18 °C, à l'aide d'une lumière UV à haute intensité, de rayons X, d'un traitement par champ électrique pulsé ou d'une pasteurisation à haute pression.
